# EUROPEAN PATENT APPLICATION

(11) **EP 4 369 218 A1**
(43) Date of publication of application: **15.05.2024**
(21) Application number: 22860385.8
(22) Date of filing: 18.08.2022
(51) Int. Cl.: G06F 16/9035

(54) **TRAINING PLAN GENERATION METHOD AND APPARATUS, ELECTRONIC DEVICE AND READABLE STORAGE MEDIUM**

(30) Priority: 23.08.2021 CN 202110972020
(71) Applicant: Huawei Technologies Co., Ltd., Shenzhen, Guangdong 518129 (CN)
(72) Inventor: JIANG, Jin, Shenzhen, Guangdong 518129 (CN); LIN, Junhao, Shenzhen, Guangdong 518129 (CN); ZHAN, Tanghai, Shenzhen, Guangdong 518129 (CN); LIU, Liang, Shenzhen, Guangdong 518129 (CN)
(74) Representative: Grünecker Patent- und Rechtsanwälte PartG mbB
(86) International application number: PCT/CN2022/113395
(87) International publication number: WO 2023/025039

(57) **Abstract**

This application is applicable to the field of data processing technologies, and provides a generation method and apparatus for a training plan, an electronic device, and a readable storage medium. The method includes: obtaining a plurality of pieces of exercise data of a user; determining, based on the plurality of pieces of exercise data, an athlete type corresponding to the user; and determining, based on the athlete type, a training direction of the user, and generating a training plan matching the training direction. According to technical solutions provided in this application, exercise data in a historical exercise process of a user may be obtained, an athlete type of the user may be determined, a training direction corresponding to training performed by the user may be identified based on the athlete type, and a training plan matching the training direction is generated, so that a training plan that adapts to an exercise capability of the user can be generated, thereby improving accuracy of the generated training plan.

## Description

This application claims priority to Chinese Patent Application No. 202110972020.5, filed with the China National Intellectual Property Administration on August 23, 2021 and entitled "GENERATION METHOD AND APPARATUS FOR TRAINING PLAN, ELECTRONIC DEVICE, AND READABLE STORAGE MEDIUM", which is incorporated herein by reference in its entirety.

### TECHNICAL FIELD

This application relates to the field of data processing technologies, and in particular, to a generation method and apparatus for a training plan, an electronic device, and a readable storage medium.

### BACKGROUND

With the improvement of living standards of people, people pay increasingly attention to physical health, and spontaneous exercise behavior of people also increases. To enhance physical fitness in a healthy and effective manner, a user can engage in training activities in a manner such as hiring a personal trainer or participating in training courses offered by a fitness institution. However, in the foregoing manner, the user needs to go to a specified training place to perform related training, and flexibility is low. To meet requirements of people for exercise at any time and implement healthy and systematic training, intelligent applications related to exercise and training emerge.

In a conventional generation technology for a training plan, a fixed training plan template is generally preset, a mapping relationship between personal information and the training plan template is established, then personal information of a user, such as a height, a weight, and an age, is collected, and a corresponding training plan is determined based on the collected personal information and the mapping relationship. In the conventional technology, a training plan is determined only based on personal information of a user and a fixed training plan template, and a single manner is used, which reduces accuracy and effectiveness of the training plan.

### SUMMARY

Embodiments of this application provide a generation method for a training plan, an adjustment method, a generation apparatus, an adjustment apparatus, an electronic device, and a readable storage medium, to effectively improve accuracy and effectiveness of a training plan.

According to a first aspect, an embodiment of this application provides a wireless connection method, including:
obtaining a plurality of pieces of exercise data of a user;
determining, based on the plurality of pieces of exercise data, an athlete type corresponding to the user; and
determining a training direction of the user based on the athlete type, and generating a training plan matching the training direction.

Implementation of this embodiment of this application has the following beneficial effects: An athlete type of the user may be determined, a training direction corresponding to training performed by the user may be identified based on the athlete type, and a training plan matching the training direction is generated, so that a training plan that adapts to an exercise capability of the user can be generated. Because an individual parameter of the user cannot directly reflect the exercise ability of the user, the training plan generated based on the individual parameter may not match the exercise ability of the user. However, the exercise data that is collected and that is in a historical exercise activity of the user can reflect an actual exercise ability of the user. The athlete type of the user is determined based on performance of the user in historical exercise, so that a matching degree between the training plan and the actual exercise ability of the user can be improved. In this way, the generated training plan can be used to guide the user to perform exercise and training more pertinently, and accuracy of the generated training plan is improved.

In a possible implementation of the first aspect, the obtained plurality of pieces of exercise data include exercise data of a plurality of exercise items, and the determining, based on the plurality of pieces of exercise data, an athlete type corresponding to the user includes:
determining, based on the exercise data associated with each exercise item, an optimal exercise score corresponding to the exercise item;
importing the optimal exercise score into a normalization algorithm corresponding to the exercise item, and determining a normalized parameter corresponding to the exercise item; and
determining the athlete type of the user based on a normalized parameter of each exercise item.

In a possible implementation of the first aspect, the determining, based on the exercise data associated with each exercise item, an optimal exercise score corresponding to the exercise item includes:
determining an ideal heart rate corresponding to the exercise item, where the ideal heart rate is specifically a corresponding heart rate of a user in a full-effort exercise state;
determining, based on an actual heart rate in the exercise data and the ideal heart rate, an intensity coefficient associated with the exercise data; and
determining, based on the intensity coefficient and an actual exercise score, the optimal exercise score corresponding to the exercise item.

In a possible implementation of the first aspect, the obtaining a plurality of pieces of exercise data of a user includes:
generating an exercise data authorization interface that includes at least one optional data source;
determining a target data source from the optional data source in response to a confirmation operation performed by the user on the exercise data authorization interface; and
obtaining the exercise data from the target data source.

In a possible implementation of the first aspect, after the determining a training direction of the user based on the athlete type, and generating a training plan matching the training direction, the method further includes:
displaying a training report corresponding to the user, where the training report includes the training plan and a descriptive paragraph about the athlete type and the training direction.

In a possible implementation of the first aspect, the determining a training direction of the user based on the athlete type, and generating a training plan matching the training direction includes:
determining, based on the training direction, phase period duration of each pre-divided training phase in a preset plan template;
determining a course category associated with the training phase, and configuring a training course that belongs to the course category for each training time in the training phase, where the training time is determined based on the phase period duration of the training phase; and
generating the training plan based on training courses corresponding to training times of all training phases.

In a possible implementation of the first aspect, the determining a course category associated with the training phase, and configuring a training course that belongs to the course category for each training time in the training phase includes:
configuring a training category library for the training phase based on the course category associated with the training phase;
determining, based on the phase period duration of the training phase and the training category library, a training course framework corresponding to the training phase, where the training course framework is used to determine the training time included in the training phase and a course difficulty level corresponding to each training time; and
selecting, for each training time from the training category library, the training course matching the course difficulty level.

In a possible implementation of the first aspect, the determining, based on the training direction, phase period duration of each pre-divided training phase in a preset plan template includes:
obtaining the plan template corresponding to a training purpose of the user, where the training purpose is preset by the user, and the plan template includes a plurality of training phases obtained through pre-division;
determining, based on the training direction, a duration proportion of each training phase; and
determining the phase period duration of the training phase based on the duration proportion of the training phase and preset total training duration.

In a possible implementation of the first aspect, before the obtaining the plan template corresponding to a training purpose of the user, the method further includes:
generating a training purpose setting interface, where the training purpose setting interface includes a purpose setting area of at least one exercise item;
determining, in response to a setting operation of the user in each purpose setting area, a target value corresponding to each exercise item; and
obtaining the training purpose based on the target value of each exercise item.

In a possible implementation of the first aspect, after the determining a training direction of the user based on the athlete type, and generating a training plan matching the training direction, the method further includes:
generating a subjective parameter collection page if it is detected that any training course in the training plan is completed, to determine, based on a feedback operation initiated by the user on the subjective parameter collection page, a subjective exercise parameter that corresponds to the any training course and that is of the user; and
adjusting the training plan based on the subjective exercise parameter, to generate an adjusted training plan.

In a possible implementation of the first aspect, before the generating a subjective parameter collection page if it is detected that any training course in the training plan is completed, the method further includes:
obtaining a corresponding training stress value when the user performs training based on the any training course; and
correspondingly, the adjusting the training plan based on the subjective exercise parameter, to generate an adjusted training plan includes:
   adjusting the training plan based on the training stress value and the subjective exercise parameter, to generate the adjusted training plan.

In a possible implementation of the first aspect, the obtaining a corresponding training stress value when the user performs training based on the any training course includes:
obtaining corresponding real-time data when the user performs training based on any training course in the training plan; and
determining, based on the real-time data, the training stress value corresponding to the training course.

In a possible implementation of the first aspect, the adjusting the training plan based on the training stress value and the subjective exercise parameter, to generate the adjusted training plan includes:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

According to a second aspect, an embodiment of this application provides a generation apparatus for a training plan, including:
an exercise data obtaining unit, configured to obtain a plurality of pieces of exercise data of a user;
an athlete type determining unit, configured to determine, based on the plurality of pieces of exercise data, an athlete type corresponding to the user; and
a training plan generation unit, configured to: determine a training direction of the user based on the athlete type, and generate a training plan matching the training direction.

In a possible implementation of the second aspect, the obtained plurality of pieces of exercise data include exercise data of a plurality of exercise items, and the athlete type determining unit includes:
an optimal exercise score determining unit, configured to determine, based on the exercise data associated with each exercise item, an optimal exercise score corresponding to the exercise item;
a normalized parameter determining unit, configured to import the optimal exercise score into a normalization algorithm corresponding to the exercise item, and determining a normalized parameter corresponding to the exercise item; and
an athlete type identification unit, configured to determine the athlete type of the user based on a normalized parameter of each exercise item.

In a possible implementation of the second aspect, the optimal exercise score determining unit includes:
an ideal heart rate determining unit, configured to determine an ideal heart rate corresponding to the exercise item, where the ideal heart rate is specifically a corresponding heart rate of a user in a full-effort exercise state;
an intensity coefficient determining unit, configured to determine, based on an actual heart rate in the exercise data and the ideal heart rate, an intensity coefficient associated with the exercise data; and
an optimal exercise score calculation unit, configured to determine, based on the intensity coefficient and an actual exercise score, the optimal exercise score corresponding to the exercise item.

In a possible implementation of the second aspect, the exercise data obtaining unit includes:
an exercise data authorization interface generation unit, configured to generate an exercise data authorization interface that includes at least one optional data source;
a confirmation operation response unit, configured to determine a target data source from the optional data source in response to a confirmation operation performed by the user on the exercise data authorization interface; and
a data obtaining unit, configured to obtain the exercise data from the target data source based on an authorization request.

In a possible implementation of the second aspect, the generation apparatus for a training plan further includes:
a training report display unit, configured to display a training report corresponding to the user, where the training report includes the training plan and a descriptive paragraph about the athlete type and the training direction.

In a possible implementation of the second aspect, the training plan generation unit includes:
a phase period duration determining unit, configured to determine, based on the training direction, phase period duration of each pre-divided training phase in a preset plan template;
a training course determining unit, configured to: determine a course category associated with the training phase, and configure a training course that belongs to the course category for each training time in the training phase, where the training time is determined based on the phase period duration of the training phase; and
a training course encapsulating unit, configured to generate the training plan based on training courses corresponding to training times of all training phases.

In a possible implementation of the second aspect, the training course determining unit includes:
a training category library configuration unit, configured to configure a training category library for the training phase based on the course category associated with the training phase;
a training course framework determining unit, configured to determine, based on the phase period duration of the training phase and the training category library, a training course framework corresponding to the training phase, where the training course framework is used to determine the training time included in the training phase and a course difficulty level corresponding to each training time; and
a training course selecting unit, configured to select, for each training time from the training category library, the training course matching the course difficulty level.

In a possible implementation of the second aspect, the phase period duration determining unit includes:
a plan template obtaining unit, configured to obtain the plan template corresponding to a training purpose of the user, where the training purpose is preset by the user, and the plan template includes a plurality of training phases obtained through pre-division;
a duration proportion determining unit, configured to determine, based on the training direction, a duration proportion of each training phase; and
a total training duration allocation unit, configured to determine the phase period duration of the training phase based on the duration proportion of the training phase and preset total training duration.

In a possible implementation of the second aspect, the generation apparatus for a training plan further includes:
a setting interface display unit, configured to generate a training purpose setting interface, where the training purpose setting interface includes a purpose setting area of at least one exercise item;
a setting operation response unit, configured to determine, in response to a setting operation of the user in each purpose setting area, a target value corresponding to each exercise item; and
a training purpose determining unit, configured to obtain the training purpose based on the target value of each exercise item.

In a possible implementation of the second aspect, the generation apparatus for a training plan further includes:
a subjective exercise parameter determining unit, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit, configured to adjust the training plan based on the subjective exercise parameter, to generate an adjusted training plan.

In a possible implementation of the second aspect, the generation apparatus for a training plan further includes:
a training stress value obtaining unit, configured to obtain a corresponding training stress value when the user performs training based on any training course in the training plan; and
the training plan adjustment unit is specifically configured to adjust the training plan based on the training stress value and the subjective exercise parameter, to generate the adjusted training plan.

In a possible implementation of the second aspect, the training stress value obtaining unit includes:
a real-time data obtaining unit, configured to obtain corresponding real-time data when the user performs training based on any training course in the training plan; and
a real-time data conversion unit, configured to determine, based on the real-time data, the training stress value corresponding to the training course.

In a possible implementation of the second aspect, the training plan adjustment unit includes:
a plan adjustment direction determining unit, configured to: if a preset adjustment trigger moment is reached, determine a plan adjustment direction based on the subjective exercise parameter and training stress values corresponding to all the training courses completed before the adjustment trigger moment; and
a training course adjustment unit, configured to adjust, based on the plan adjustment direction, a training course that is in the training plan and that is after the adjustment trigger moment, to obtain the adjusted training plan.

According to a third aspect, an embodiment of this application provides a generation method for a training plan, including:
determining, based on a training direction of a user, phase period duration of each pre-divided training phase in a preset plan template;
determining a course category associated with the training phase, and configuring a training course that belongs to the course category for each training time in the training phase, where the training time is determined based on the phase period duration of the training phase; and
generating the training plan based on training courses corresponding to training times of all training phases.

In a possible implementation of the third aspect, the determining a course category associated with the training phase, and configuring a training course that belongs to the course category for each training time in the training phase includes:
configuring a training category library for the training phase based on the course category associated with the training phase;
determining, based on the phase period duration of the training phase and the training category library, a training course framework corresponding to the training phase, where the training course framework is used to determine the training time included in the training phase and a course difficulty level corresponding to each training time; and
selecting, for each training time from the training category library, the training course matching the course difficulty level.

In a possible implementation of the third aspect, the determining, based on a training direction of a user, phase period duration of each pre-divided training phase in a preset plan template includes:
obtaining the plan template corresponding to a training purpose of the user, where the training purpose is preset by the user, and the plan template includes a plurality of training phases obtained through pre-division;
determining, based on the training direction, a duration proportion of each training phase; and
determining the phase period duration of the training phase based on the duration proportion of the training phase and preset total training duration.

In a possible implementation of the third aspect, before the obtaining the plan template corresponding to a training purpose of the user, the method further includes:
generating a training purpose setting interface, where the training purpose setting interface includes a purpose setting area of at least one exercise item;
determining, in response to a setting operation of the user in each purpose setting area, a target value corresponding to each exercise item; and
obtaining the training purpose based on the target value of each exercise item.

In a possible implementation of the third aspect, after the generating the training plan based on training courses corresponding to training times of all the training phases, the method further includes:
generating a subjective parameter collection page if it is detected that any training course in the training plan is completed, to determine, based on a feedback operation initiated by the user on the subjective parameter collection page, a subjective exercise parameter that corresponds to the any training course and that is of the user; and
adjusting the training plan based on the subjective exercise parameter, to generate an adjusted training plan.

In a possible implementation of the third aspect, before the generating a subjective parameter collection page if it is detected that any training course is completed, the method further includes:
obtaining a corresponding training stress value when the user performs training based on the any training course; and
correspondingly, the adjusting the training plan based on the subjective exercise parameter, to generate an adjusted training plan includes:
   adjusting the training plan based on the training stress value and the subjective exercise parameter, to generate the adjusted training plan.

In a possible implementation of the third aspect, the obtaining a corresponding training stress value when the user performs training based on the any training course includes:
obtaining corresponding real-time data when the user performs training based on any training course in the training plan; and
determining, based on the real-time data, the training stress value corresponding to the training course.

In a possible implementation of the third aspect, the adjusting the training plan based on the training stress value and the subjective exercise parameter, to generate the adjusted training plan includes:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

According to a fourth aspect, an embodiment of this application provides a generation apparatus for a training plan, including:
a phase period duration determining unit, configured to determine, based on a training direction of a user, phase period duration of each pre-divided training phase in a preset plan template;
a training course determining unit, configured to: determine a course category associated with the training phase, and configure a training course that belongs to the course category for each training time in the training phase, where the training time is determined based on the phase period duration of the training phase; and
a training course encapsulating unit, configured to generate the training plan based on training courses corresponding to training times of all training phases.

In a possible implementation of the fourth aspect, the training course determining unit includes:
a training category library configuration unit, configured to configure a training category library for the training phase based on the course category associated with the training phase;
a training course framework determining unit, configured to determine, based on the phase period duration of the training phase and the training category library, a training course framework corresponding to the training phase, where the training course framework is used to determine the training time included in the training phase and a course difficulty level corresponding to each training time; and
a training course selecting unit, configured to select, for each training time from the training category library, the training course matching the course difficulty level.

In a possible implementation of the fourth aspect, the phase period duration determining unit includes:
a plan template obtaining unit, configured to obtain the plan template corresponding to a training purpose of the user, where the training purpose is preset by the user, and the plan template includes a plurality of training phases obtained through pre-division;
a duration proportion determining unit, configured to determine, based on the training direction, a duration proportion of each training phase; and
a total training duration allocation unit, configured to determine the phase period duration of the training phase based on the duration proportion of the training phase and preset total training duration.

In a possible implementation of the fourth aspect, the generation apparatus for a training plan further includes:
a setting interface display unit, configured to generate a training purpose setting interface, where the training purpose setting interface includes a purpose setting area of at least one exercise item;
a setting operation response unit, configured to determine, in response to a setting operation of the user in each purpose setting area, a target value corresponding to each exercise item; and
a training purpose determining unit, configured to obtain the training purpose based on the target value of each exercise item.

In a possible implementation of the fourth aspect, the generation apparatus for a training plan further includes:
a subjective exercise parameter determining unit, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit, configured to adjust the training plan based on the subjective exercise parameter, to generate an adjusted training plan.

In a possible implementation of the fourth aspect, the generation apparatus for a training plan further includes:
a training stress value obtaining unit, configured to obtain a corresponding training stress value when the user performs training based on any training course in the training plan; and
the training plan adjustment unit is specifically configured to adjust the training plan based on the training stress value and the subjective exercise parameter, to generate the adjusted training plan.

In a possible implementation of the fourth aspect, the training stress value obtaining unit includes:
a real-time data obtaining unit, configured to obtain corresponding real-time data when the user performs training based on any training course in the training plan; and
a real-time data conversion unit, configured to determine, based on the real-time data, the training stress value corresponding to the training course.

In a possible implementation of the fourth aspect, the training plan adjustment unit includes:
a plan adjustment direction determining unit, configured to: if a preset adjustment trigger moment is reached, determine a plan adjustment direction based on the subjective exercise parameter and training stress values corresponding to all the training courses completed before the adjustment trigger moment; and
a training course adjustment unit, configured to adjust, based on the plan adjustment direction, a training course that is in the training plan and that is after the adjustment trigger moment, to obtain the adjusted training plan.

According to a fifth aspect, an embodiment of this application provides an adjustment method for a training plan, including:
generating a subjective parameter collection page if it is detected that any training course in a training plan is completed, to determine, based on a feedback operation initiated by a user on the subjective parameter collection page, a subjective exercise parameter that corresponds to the any training course and that is of the user; and
adjusting the training plan based on the subjective exercise parameter, to generate an adjusted training plan.

In a possible implementation of the fifth aspect, before the generating a subjective parameter collection page if it is detected that any training course in the training plan is completed, the method further includes:
obtaining a corresponding training stress value when the user performs training based on the any training course; and
correspondingly, the adjusting the training plan based on the subjective exercise parameter, to generate an adjusted training plan includes:
   adjusting the training plan based on the training stress value and the subjective exercise parameter, to generate the adjusted training plan.

In a possible implementation of the fifth aspect, the obtaining a corresponding training stress value when the user performs training based on the any training course includes:
obtaining corresponding real-time data when the user performs training based on any training course in the training plan; and
determining, based on the real-time data, the training stress value corresponding to the training course.

In a possible implementation of the fifth aspect, the adjusting the training plan based on the training stress value and the subjective exercise parameter, to generate the adjusted training plan includes:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

According to a sixth aspect, an embodiment of this application provides an adjustment apparatus for a training plan, including:
a subjective exercise parameter determining unit, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit, configured to adjust the training plan based on the subjective exercise parameter, to generate an adjusted training plan.

In a possible implementation of the sixth aspect, the adjustment apparatus for a training plan further includes:
a training stress value obtaining unit, configured to obtain a corresponding training stress value when the user performs training based on any training course in the training plan; and
the training plan adjustment unit is specifically configured to adjust the training plan based on the training stress value and the subjective exercise parameter, to generate the adjusted training plan.

In a possible implementation of the sixth aspect, the training stress value obtaining unit includes:
a real-time data obtaining unit, configured to obtain corresponding real-time data when the user performs training based on any training course in the training plan; and
a real-time data conversion unit, configured to determine, based on the real-time data, the training stress value corresponding to the training course.

In a possible implementation of the sixth aspect, the training plan adjustment unit includes:
a plan adjustment direction determining unit, configured to: if a preset adjustment trigger moment is reached, determine a plan adjustment direction based on the subjective exercise parameter and training stress values corresponding to all the training courses completed before the adjustment trigger moment; and
a training course adjustment unit, configured to adjust, based on the plan adjustment direction, a training course that is in the training plan and that is after the adjustment trigger moment, to obtain the adjusted training plan.

According to a seventh aspect, an embodiment of this application provides an electronic device, a memory, a processor, and a computer program that is stored in the memory and that can be run on the processor. When executing the computer program, the processor implements the generation method for a training plan according to any one of the first aspect or the third aspect, or the adjustment method for a training plan according to any one of the fifth aspect.

According to an eighth aspect, an embodiment of this application provides a computer-readable storage medium, where the computer-readable storage medium stores a computer program, and when the computer program is executed by a processor, the generation method for a training plan according to any one of the first aspect or the third aspect or the adjustment method for a training plan according to any one of the fifth aspect is implemented.

According to a ninth aspect, an embodiment of this application provides a computer program product. When the computer program product runs on an electronic device, the electronic device performs the generation method for a training plan according to any one of the first aspect or the third aspect, or the adjustment method for a training plan according to any one of the fifth aspect.

According to a tenth aspect, an embodiment of this application provides a chip system, including a processor, where the processor is coupled to a memory, and the processor executes a computer program stored in the memory, to implement the generation method for a training plan according to any one of the first aspect or the third aspect, or the adjustment method for a training plan according to any one of the fifth aspect.

It may be understood that for beneficial effects of the second aspect to the tenth aspect, refer to the related description in the first aspect. Details are not described herein again.

### BRIEF DESCRIPTION OF DRAWINGS

FIG. 1 is a schematic diagram of conventional training plan generation;
FIG. 2A and FIG. 2B are an implementation flowchart of a generation method for a training plan according to an embodiment of this application;
FIG. 3A and FIG. 3B are a schematic diagram of obtaining exercise data according to an embodiment of this application;
FIG. 4(a) to FIG. 4(c) are schematic diagrams of information input interfaces according to an embodiment of this application;
FIG. 5(a) to FIG. 5(g) are a schematic diagram of a process of authorizing exercise data according to an embodiment of this application;
FIG. 6 is a schematic diagram of a running exercise scenario according to an embodiment of this application;
FIG. 7 is a schematic diagram of merging exercise data according to an embodiment of this application;
FIG. 8 is a schematic diagram of calculating an optimal exercise score according to an embodiment of this application;
FIG. 9 is a schematic diagram of a normalization algorithm according to an embodiment of this application;
FIG. 10 is a diagram of a correspondence between athlete types according to an embodiment of this application;
FIG. 11 is a schematic diagram of a training plan display process according to an embodiment of this application;
FIG. 12 is a schematic diagram of querying a training plan according to an embodiment of this application;
FIG. 13A and FIG. 13B are an implementation flowchart of a generation method for a training plan according to another embodiment of this application;
FIG. 14 is a schematic diagram of division of training phases according to an embodiment of this application;
FIG. 15(a) to FIG. 15(c) are schematic diagrams of setting total training duration according to an embodiment of this application;
FIG. 16 is a schematic diagram of selecting a training course according to an embodiment of this application;
FIG. 17 is a schematic diagram of a training calendar interface according to an embodiment of this application;
FIG. 18A and FIG. 18B are an implementation flowchart of an adjustment method for a training plan according to an embodiment of this application;
FIG. 19 is a schematic diagram of a subjective parameter collection page according to an embodiment of this application;
FIG. 20 is a schematic diagram of a training plan adjustment direction according to an embodiment of this application;
FIG. 21 is a schematic diagram of adjustment of a training course according to an embodiment of this application;
FIG. 22(a) to FIG. 22(c) are schematic diagrams of adjustment of a training plan according to an embodiment of this application;
FIG. 23 is a block diagram of a structure of a generation apparatus for a training plan according to an embodiment of this application;
FIG. 24 is a block diagram of a structure of a generation apparatus for a training plan according to another embodiment of this application;
FIG. 25 is a block diagram of a structure of a generation apparatus for a training plan according to an embodiment of this application;
FIG. 26 is a block diagram of a structure of an adjustment apparatus for a training plan according to an embodiment of this application;
FIG. 27 is a schematic diagram of a hardware structure of an electronic device according to an embodiment of this application; and
FIG. 28 is a block diagram of a software structure of an electronic device according to an embodiment of this application.

### DESCRIPTION OF EMBODIMENTS

In the following descriptions, for illustration instead of limitation, specific details such as a particular system structure and a technology are provided to make a thorough understanding of embodiments of this application. However, a person skilled in the art should know that this application may be implemented in other embodiments without these specific details. In other cases, detailed descriptions of well-known systems, apparatuses, circuits, and methods are omitted, so that this application is described without being obscured by unnecessary details.

It should be understood that, when used in the specification and the appended claims of this application, the term "include" indicates presence of the described features, entireties, steps, operations, elements, and/or components, but does not exclude presence or addition of one or more other features, entireties, steps, operations, elements, components, and/or sets thereof.

It should also be understood that the term "and/or" used in the specification and the appended claims of this application refers to any combination and all possible combinations of one or more associated listed items, and includes these combinations.

As used in the specification and the appended claims of this application, according to the context, the term "if" may be interpreted as "when" or "once" or "in response to determining" or "in response to detecting". Likewise, the phrase "if it is determined that" or "if [a described condition or event] is detected" may be interpreted as a meaning of "once it is determined that" or "in response to determining" or "once [a described condition or event] is detected" or "in response to detecting [a described condition or event]" depending on the context.

In addition, in the descriptions of the specification and claims of this application, the terms "first", "second", "third", and the like are merely intended for a purpose of differentiated description, but shall not be understood as an indication or an implication of relative importance.

Reference to "an embodiment", "some embodiments", or the like described in the specification of this application indicates that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

As people pay more attention to physical health, how to effectively and systematically perform training exercises has become one of problems that need to be resolved urgently. In particular, a concept of mass sports continuously gains popularity, and a boundary between amateur and professional athletes in the sports field is becoming increasingly blurred. People are not only limited to physical fitness, but also hope to participate in sports-related competitions. Achieving the above purposes is not separated from designation of professional training plans. The marathon exercise is used as an example. Because the marathon has characteristics such as a long distance, large physical energy consumption, and long duration, for a user who has not experienced long-distance running, the marathon has a specific risk, and is a movement with a high entry threshold. If a user plans to participate in a marathon competition, the used need to perform targeted training with specific time in advance. In this case, the user needs to be trained and guided in a reliable and convenient manner, and needs to arrange a corresponding training plan.

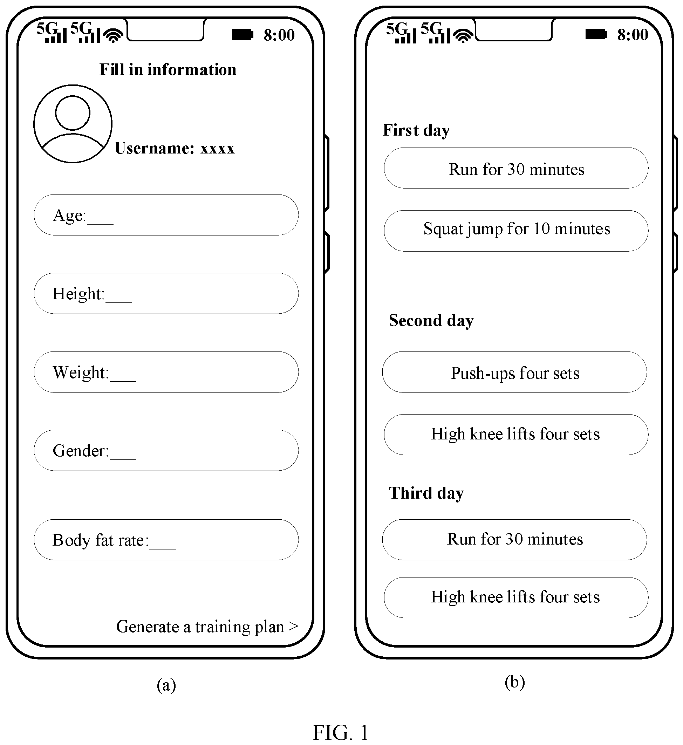

A conventional training plan generation technology may mainly include the following two types:
Manner 1: A training plan is generated by collecting an individual parameter of a user. The user may install, on a smartphone of the user, an application for generating a training plan. By running the application for generating a training plan, an input interface for inputting an individual parameter of the user may be generated. For example, FIG. 1 is a schematic diagram of conventional training plan generation. Refer to (a) in FIG. 1. In an input interface for inputting an individual parameter, the user needs to fill in the following parameter items: an age, a height, a weight, a gender, and a body fat rate, and select, based on related data filled in by the user, a training course corresponding to physical quality of the user, to generate a corresponding training plan, as shown in (b) in FIG. 1. It can be learned that, when a training plan is generated by using an individual parameter of a user, the corresponding training plan can be generated by collecting individual parameters of different ages, heights, weights, genders, and the like, performing limited arrangement and combination, and associating different combinations with corresponding training courses.

However, in this manner, only the individual parameter of the user is considered to determine the corresponding training plan, and accuracy of the generated training plan is low. In addition to individual parameters of users, determining a difference between exercise capabilities users is related to a plurality of factors such as a user exercise intention, an exercise purpose, and proficiency in a related exercise. In the foregoing manner, only differences between individual parameters are considered, and factors for determining the training plan are too singular. Consequently, the user cannot be effectively and accurately guided to perform training.

Manner 2: Whether a training plan needs to be adjusted is determined by collecting whether a biological feature indicator of a user in an exercise process exceeds a human body limit value. Manner 2 is a further optimization of manner 1. A user may enter an individual parameter on a smartphone, generate a corresponding training plan by using a related application installed on the smartphone, and perform training based on the training plan. In a training process of the user, a biological feature parameter used when the user performs training based on the training plan may be obtained by wearing a smartwatch or a smart band. If it is detected that the biological feature parameter of the user exceeds a human body limit value, for example, when running training is performed, it is detected that a heart rate of the user exceeds a normal heart rate range of a human body, in this case, it is determined that the training plan exceeds a bearing range of the user, and a created training plan is adjusted. In this manner, after an exception occurs in a user training process, the training plan can be optimized and adjusted, to improve a matching degree between the training plan and the user.

In this manner, a biometric feature indicator of the user in an exercise process can be collected, to implement adjustment and optimization on a generated training plan, to improve a degree of conformity between the training plan and the user. However, an adjustment triggering condition is that adjustment is performed only when the biometric feature indicator of the user exceeds a human body limit. Generally, when the biometric feature indicator of the user exceeds the human body limit, there is a health risk. Therefore, it can be learned that it is difficult to initiate the optimization adjustment, and if a triggering threshold is reduced from a human body threshold to a preset threshold, the training plan may also be frequently adjusted. This reduces stability of the training plan, and is unfavorable for the user to arrange training exercise.

It can be learned that, in a conventional training plan generation technology, a degree of conformity between a training plan and a user is low, accuracy of the training plan is low, and optimization timeliness and plan stability cannot be considered for adjustment and optimization of the training plan. Therefore, to resolve a defect of low accuracy of the training plan, this application provides a generation method for a training plan. A training focus of a user is determined, and then a training course matching the training focus of the user is obtained from a course database, to generate a training plan used by the user, to improve accuracy of the training plan. In addition, in a process in which the user performs training based on the training plan, a biometric feature indicator of the user and a subjective perception indicator of the user in an exercise process are obtained, the training plan is optimized by using the foregoing two types of parameters, thereby improving accuracy of an optimization operation, and reducing occurrence of invalid optimization.

### Embodiment 1

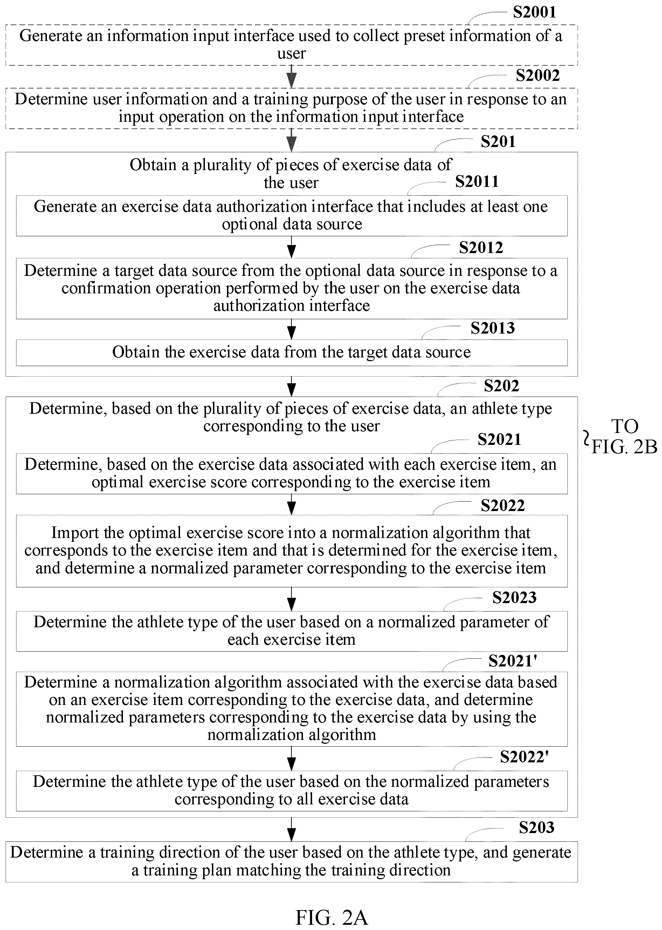

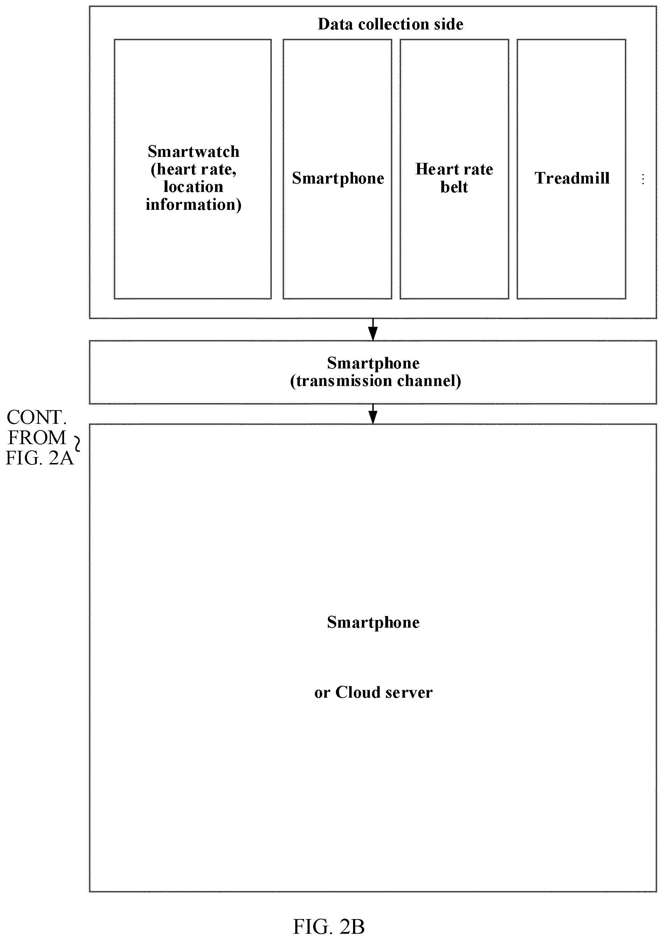

In this embodiment of this application, a procedure is executed by an electronic device. The electronic device includes but is not limited to a device that can execute a training plan generation task, such as a computer, a smartphone, a notebook computer, a tablet computer, a wearable intelligent device (such as a smart necklace, a smart watch, and a smart band), and a cloud server. FIG. 2A and FIG. 2B are an implementation flowchart of a generation method for a training plan according to an embodiment of this application. Details are as follows:
In S201, an electronic device obtains a plurality of pieces of exercise data of a user.

In this embodiment, after completing one exercise, exercise data related to the current exercise may be generated. The exercise data may record one or a combination of the following: an exercise type, exercise duration, a peak heart rate, and the like. If an exercise type of the exercise is running, the exercise data may include an exercise distance, a vertical climbing height, and the like. If the exercise type of the exercise is high jump, the exercise data may include a maximum height, a success ratio, and the like. If the exercise type of the exercise is a cycling type, the exercise data may include a cycling distance, a track type, and the like. Therefore, a parameter item included in exercise data is determined based on a corresponding exercise item.

In this embodiment, in an exercise process, the user may obtain the exercise data by using a data collection apparatus. The data collection apparatus for collecting the exercise data of the user may be the electronic device in this embodiment, or may be another device other than the electronic device. In this case, the electronic device may obtain the exercise data of the user from another device.

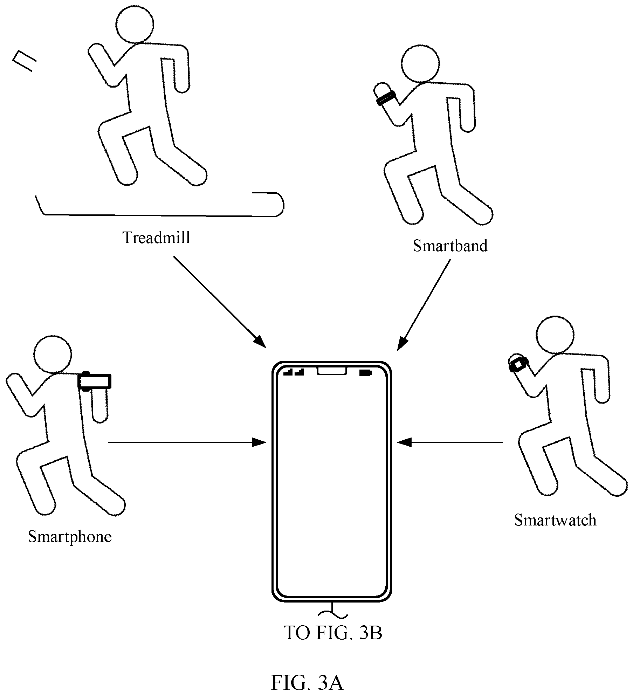

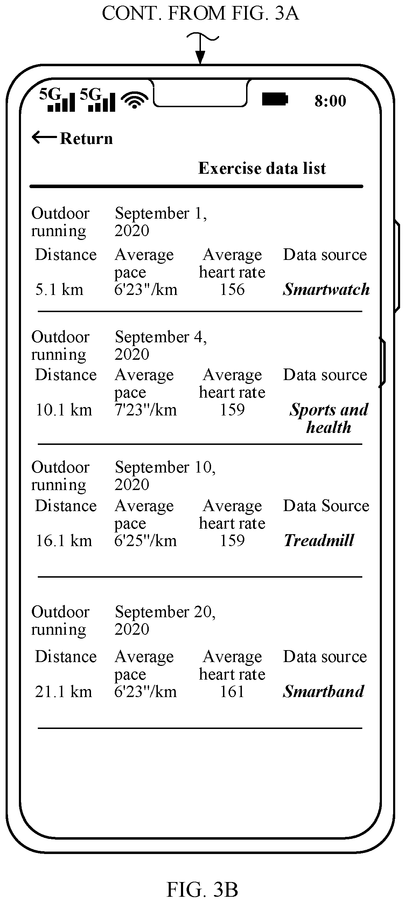

A running exercise is used as an example for description. For example, FIG. 3A and FIG. 3B a schematic diagram of obtaining exercise data according to an embodiment of this application. Refer to FIG. 3(A) and FIG. 3(B). The electronic device is specifically a smartphone of a user. A health application may be configured in the smartphone. The user may record related data of the user in an exercise process by using the health application in the exercise process, and after an exercise is completed, the smartphone may generate exercise data about the current run. The electronic device may alternatively obtain the exercise data of the user from another device. For example, when the user wears a smartwatch to perform exercise, the smartwatch may also record corresponding exercise data. Similarly, devices such as a treadmill, a smart band, a smart necklace, and a heart rate band may also collect corresponding exercise data when the user runs. In this case, the smartphone (namely, the electronic device in this embodiment) may establish a communication connection to the foregoing electronic device, and obtain the exercise data of the user from another electronic device. Certainly, the electronic device may further obtain the exercise data of the user from another third-party device. A corresponding exercise data list may be generated on the electronic device based on the obtained exercise data. The exercise data list includes information such as a data source, an exercise time, an exercise item, an exercise distance, and exercise duration corresponding to each piece of exercise data.

Further, in another embodiment of this application, before S201, the method may further include the following steps:
In S2001, an information input interface used to collect preset information of a user is generated, where the preset information includes user information and a training purpose.

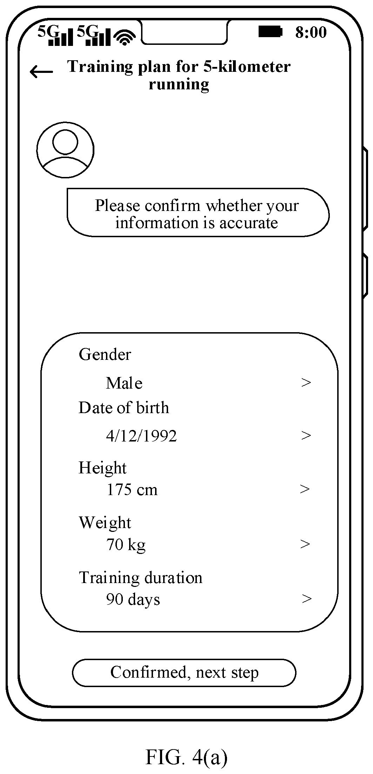

In this embodiment, before obtaining the exercise data, the electronic device may first obtain the preset information, and generate the information input interface used to collect the preset information. The information input interface is specifically used to obtain two types of preset information of the user: user information and a training purpose. For example, FIG. 4(a) to FIG. 4(c) are schematic diagrams of information input interfaces according to an embodiment of this application. FIG. 4(a) is used to collect user information of a user. The user information may include an age, a height, a gender, a weight, and training duration of the user.

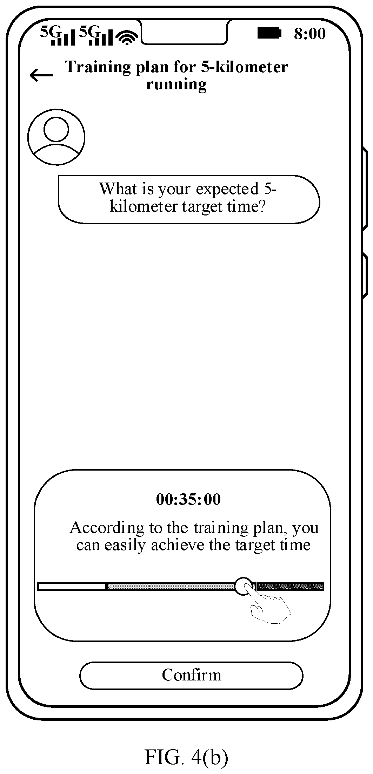

Further, in another embodiment of this application, S2001 may further include: generating a training purpose setting interface, where the training purpose setting interface includes a purpose setting area of at least one exercise item; determining a target value corresponding to each exercise item in response to a setting operation of the user in each purpose setting area; and obtaining the training purpose based on the target value of each exercise item. An interface in FIG. 4(b) is used to collect the training purpose of the user. The training purpose may be exercise scores corresponding to different training items, for example, a target time of running at 5 kilometers. The user may drag a sliding control in the setting interface to adjust a target value of a corresponding exercise item. It should be noted that, in this example, a training plan that the user needs to create is a training plan for running at 5 kilometers. Certainly, in another scenario, different training plans may be created.

In S2002, the user information and the training purpose of the user are determined in response to an input operation on the information input interface, where the user information is used to determine an athlete type of the user, and the training purpose is used to determine a training plan of the user.

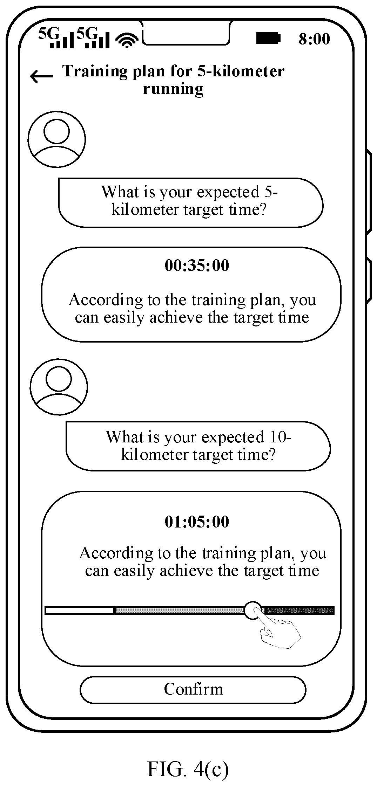

In this embodiment, the user may input the corresponding user information and the training purpose on the information input interface displayed by the electronic device, and determine the athlete type by using the user information in a subsequent operation. The training purpose may be used to formulate a training plan of the user. In a possible implementation, the training purpose may include exercise scores corresponding to different training items. In this case, after setting an exercise score of a training item, the user may be prompted to enter an exercise score of a next item, for example, FIG. 4(c). When exercise scores of all exercise items are set, an operation of a next step is performed.

Further, in another embodiment of this application, when the electronic device obtains the exercise data from the another device, authorization confirmation of the user needs to be obtained, so that the exercise data can be obtained from a data source. In this case, S201 may specifically include the following steps:
In S2011, an exercise data authorization interface that includes at least one optional data source is generated.

In this embodiment, before obtaining the exercise data from another device, the electronic device first needs to perform authorization and authentication on an operation of obtaining the exercise data. Therefore, the electronic device may generate an exercise data authorization interface, and the exercise data authorization interface includes at least one optional data source that can obtain the exercise data of the user. The optional data source may be another device other than the electronic device, or may be an application installed in the electronic device. For example, a "health" application is installed in the electronic device. The application may record exercise data of the user during exercise of the user, and encapsulate the exercise data by using a preset format. Because the exercise data is a type of privacy data of a user, obtaining the privacy data of the user generally requires authorization permission of the user. Therefore, the electronic device may generate the exercise data authorization interface, to prompt the user to perform authorization and authentication on obtaining the exercise data.

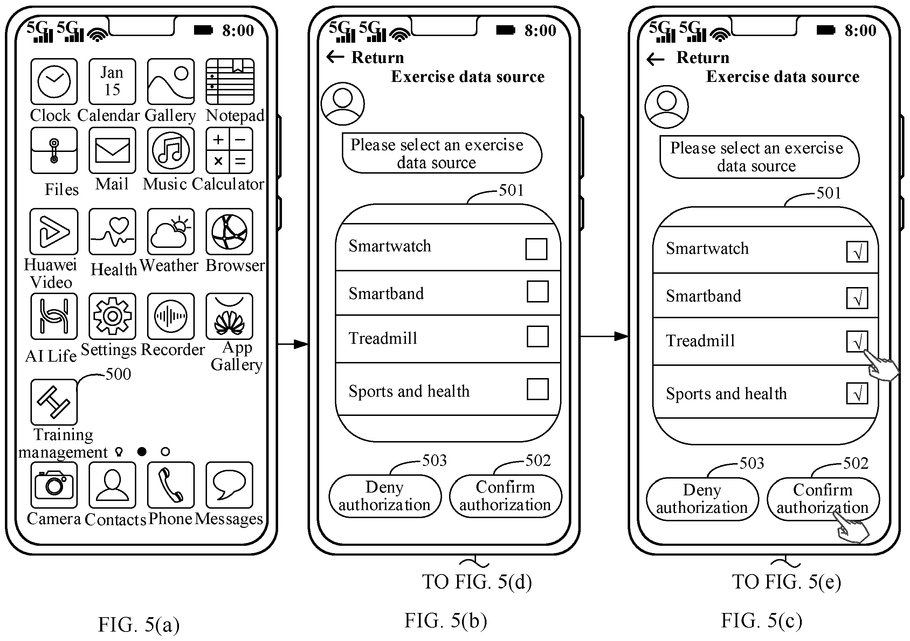

For example, FIG. 5(a) to FIG. 5(g) are a schematic diagram of a process of authorizing exercise data according to an embodiment of this application. FIG. 5(a) is a schematic diagram of a home screen displayed by an electronic device. A user may tap an application icon 500 corresponding to "training management" on the home screen, to start a training plan generation process. Because a first step of generating a training plan is to obtain the exercise data of the user, when a "training management" application is started for the first time, or when the "training management" application detects that no training plan is generated, the exercise data authorization interface may be generated, as shown in FIG. 5(b). The exercise data authorization interface includes a data source list 501, and at least one optional data source is marked in the data source list 501, for example, four optional data sources "smartwatch", "smart band", "treadmill", and "health" displayed in FIG. 5(b). The first three optional data sources need to be obtained from another device other than the electronic device, and the "health" may be obtained from a database associated with an application installed locally. In addition to the data source list 501 corresponding to the optional data source, the exercise data authorization interface further includes a "confirm authorization" control 502 and a "deny authorization" control 503.

In a possible implementation, a wireless communication module may be configured in the electronic device. The wireless communication module may be a wireless high-fidelity Wi-Fi module, a Bluetooth module, a Bluetooth low energy module, or the like. The electronic device may search for a wireless signal in a scenario by using the wireless communication module, parse a wireless signal to obtain a connectable device in the scenario, and add each connectable device to the foregoing data source list.

In a possible implementation, the user is associated with a user account. The user account records a plurality of optional devices, and each optional device stores exercise data of the user. The user may log in to a same user account on electronic devices. In this case, the electronic device may display each associated optional device in the foregoing optional device list based on device information associated with the user account (that is, information that records another device that logs in to the user account), so that the user selects a device of a target data source from the associated another device.

In S2012, a target data source from the optional data source is determined in response to a confirmation operation performed by the user on the exercise data authorization interface.

In this embodiment, if the user agrees that the electronic device obtains the exercise data, the user may select, from the foregoing optional data source, a data source that can be authorized, and after the selection is completed, perform confirmation. The electronic device may use the optional data source selected by the user as the target data source, that is, may obtain the exercise data of the user from the target data source.

Refer to FIG. 5(b). In the exercise data authorization interface, an initial state of each optional data source is a to-be-selected state. The user may select, from the optional data source, a data source that can be obtained by the electronic device, and tap a corresponding area of the optional data source in the exercise data authorization interface. The electronic device identifies the tap operation as a selection operation, and switches a state of the optional data source to a selected state, as shown in FIG. 5(c). For example, the user selects all available data sources as the target data source. If the electronic device detects that the user taps the "confirm authorization" control 502, the electronic device identifies that the user initiates the foregoing confirmation operation, and determines the target data source based on a selection status of each optional data source when the confirmation operation is initiated.

In a possible implementation, the initial state of each optional data source is a selected state, that is, the generated exercise data authorization interface is shown in FIG. 5(c). The user may select an optional data source that is not authorized, to change a status of the data source from a selected state to a to-be-selected state, and after the selection is completed, tap the "confirm authorization" control 502.

In a possible implementation, maximum wait duration may be configured on the exercise data authorization interface. If the electronic device does not receive, within the maximum wait duration, a confirmation operation initiated by the user, the electronic device identifies that the user refuses to authorize to obtain the exercise data.

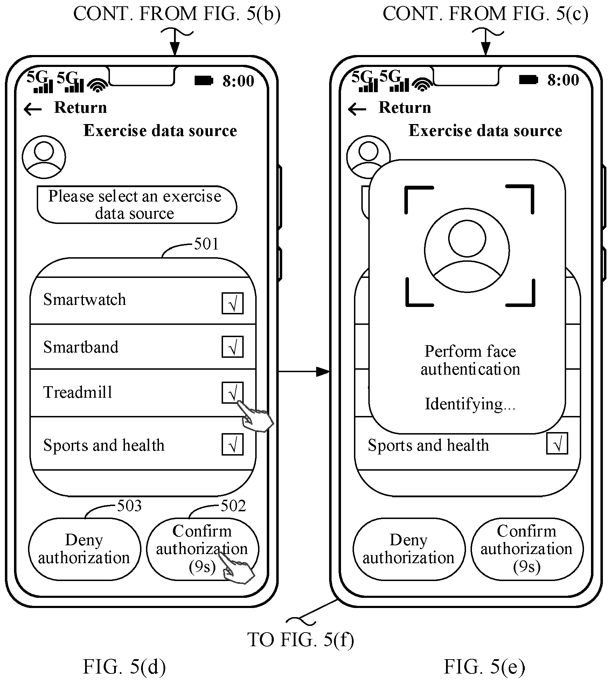

In a possible implementation, maximum wait duration may be configured on the exercise data authorization interface. If the electronic device does not receive, within the maximum wait duration, a confirmation operation initiated by the user, the target data source is determined based on a default setting, for example, all optional data sources are used as the target data source. As shown in FIG. 5(d), the "confirm authorization" control 502 displays a remaining time, for example, "9s". If no confirmation operation of the user is received within 9s, it is identified that the user uses all optional data sources as the target data sources by default.

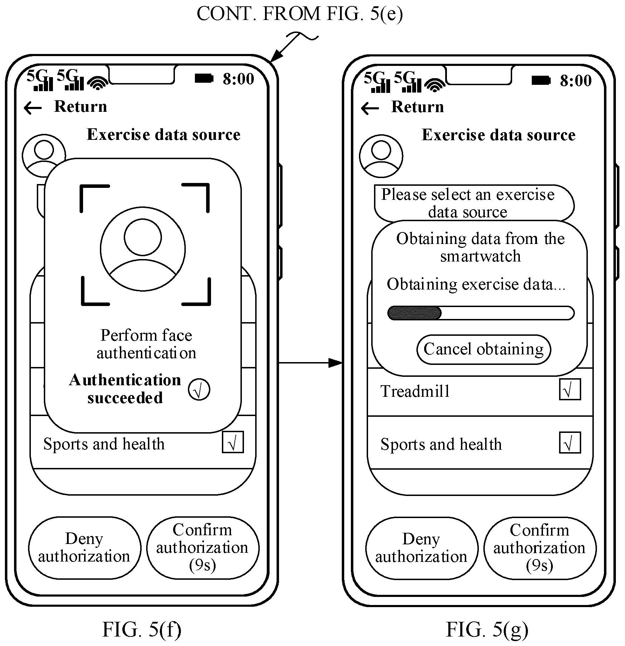

In a possible implementation, after receiving the confirmation operation initiated by the user and before generating an authorization request, the electronic device may authenticate an identity of the user. For example, the electronic device may generate authentication prompt information, as shown in FIG. 5(e), to require face authentication on the user, to determine that a user who initiates the current confirmation operation is a user to which the exercise data belongs. If the authentication succeeds, prompt information indicating that the authentication succeeds may be generated, as shown in FIG. 5(f). It is identified that the user agrees to authorize to obtain the exercise data from the target data source, and an operation in S2013 is performed.

In S2013, the exercise data is obtained from the target data source.

In this embodiment, the electronic device may directly obtain the exercise data from the target data source after identifying that the user agrees to obtain the exercise data. Optionally, after receiving the confirmation operation initiated by the user, the electronic device may generate a corresponding authorization request, obtain data obtaining permission of the target data source by using the authorization request, and then obtain the exercise data of the user from the target data source. Still refer to FIG. 5(g). In a process in which the electronic device obtains the exercise data from the target data source, an obtaining progress prompt box may be generated, so that the user determines a target data source from which the exercise data is currently obtained and an obtaining progress of the exercise data.

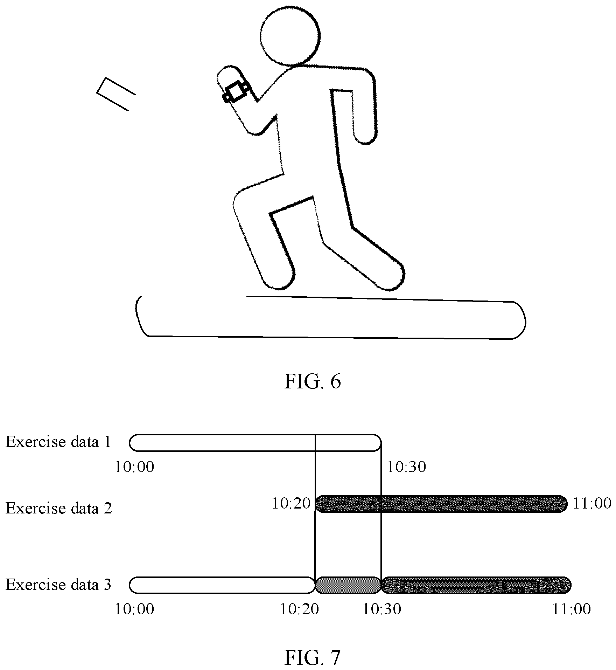

In a possible implementation, after obtaining the exercise data from each target data source, the electronic device may sort and merge the exercise data, and merge exercise data that belongs to a same exercise. For example, FIG. 6 is a schematic diagram of a running exercise scenario according to an embodiment of this application. When performing a running exercise, a user may exercise on a treadmill. In this case, the user wears a smartwatch. In this case, both the smartwatch of the user and the treadmill record exercise data of the running behavior. When obtaining the exercise data from the two data sources, the electronic device obtains two different pieces of exercise data, that is, exercise data 1 from the smartwatch and exercise data 2 from the treadmill. In this case, the electronic device may merge the exercise data 1 and the exercise data 2. A specific merging manner may be: identifying a start time and an end time of each piece of exercise data, and determining an exercise time period corresponding to the exercise data; and if there is an overlapping time period in exercise time periods corresponding to two pieces of exercise data, merging the two pieces of exercise data. For example, if an exercise time period of the exercise data 1 includes an exercise time period of the exercise data 2, the exercise data 1 is retained, and the exercise data 2 is deleted. If the exercise time periods of the two pieces of exercise data partially overlap, overlapping parts in the two pieces of exercise data are merged, and non-overlapping parts are spliced according to a sequence relationship between the non-overlapping parts and the overlapping parts, to obtain merged exercise data.

For example, FIG. 7 is a schematic diagram of merging exercise data according to an embodiment of this application. An exercise time period corresponding to the exercise data 1 obtained by the electronic device is 10:00 to 10:30, and an exercise time period corresponding to the exercise data 2 is 10:20 to 11:00. It can be learned that in the exercise time period 10:20 to 10:30, the exercise data 1 and the exercise data 2 overlap. When merging the two pieces of exercise data, the electronic device obtains a merged data segment based on an exercise data average value of the exercise data 1 and the exercise data 2 in the overlapping time period (10:20 to 10:30), and splice and merge the merged data segment and the remaining data segments accordingly, to obtain exercise data 3.

In a possible implementation, the electronic device obtains exercise data of a target user in a plurality of different exercise items. The electronic device may perform more accurate evaluation on an overall exercise capability of the user by using exercise data of a plurality of different exercise items, to improve accuracy of identifying an athlete type of the user.

For example, if the user needs to generate a running-type training plan, the exercise data of the exercise item may be specifically exercise data corresponding to different running distances, for example, running data of 5 kilometers, running data of 10 kilometers, running data of a half marathon (21.09 kilometers), and running data of a full marathon (42.19 kilometers). Certainly, a specific running distance may be set based on an actual situation, and a difference between exercise items is not limited herein.

In a possible implementation, different exercise items may belong to a same exercise type. As described above, the exercise data in a plurality of different exercise items may be exercise data in different running distances. Different exercise items may belong to different exercise types. For example, exercise items that need to be obtained are a long-distance running item, a swimming item, a high jump item, a long jump item, and the like. Different exercise items may reflect different capabilities of the user, and can also determine an athlete type of the user. For example, a long-distance running item may determine endurance of the user, and a high jump and a long jump may determine explosive power of the user. Therefore, exercise capabilities corresponding to the user in different dimensions are determined by using different exercise items.

In a possible implementation, if the electronic device does not obtain the exercise data of the user, that is, the user does not record the exercise data by using the electronic device or another device before generating a training plan, that is, there is no corresponding data source, the electronic device may identify that the athlete type corresponding to the user is a preset type, for example, an entry type.

In S202, an athlete type corresponding to the user is determined based on the plurality of pieces of exercise data.

In this embodiment, after obtaining the plurality of pieces of exercise data of the user, the electronic device may determine the exercise capability of the user by using the plurality of pieces of exercise data. Running exercise is used as an example for description. If the plurality of pieces of exercise data of the user are exercise data of a sprint type, and an average pace of each sprint is greater than a preset average value, it may be inferred that the user likes sprinting and is good at sprinting, and the athlete type of the user may be determined as the sprint type. It can be learned that the electronic device may determine, by using the plurality of pieces of exercise data, the athlete type corresponding to the user.

In this embodiment, the foregoing athlete type may be classified according to different manners. For example, the athlete type may be classified based on a degree of proficiency in an exercise, and may be classified into a novice type, a balanced type, an elite type, and the like; or the athlete type may be classified based on an exercise item that the user is good at, for example, may be classified into a sprint type, a long-distance running type, or the like; or may be classified into a running type, a basketball type, a football type, a swimming type, and the like.

In this embodiment, a manner in which the electronic device determines the athlete type based on the plurality of pieces of exercise data may be: determining, based on an exercise item corresponding to the exercise data, a capability dimension (such as muscle endurance, explosive power, and skill) that is associated with the exercise data and that is related to an exercise capability, determining an exercise time, a heart rate value, and the like that correspond to the exercise data, and determining a dimension score corresponding to each capability dimension. The foregoing processing is performed on all exercise data, to determine a dimension score of each piece of exercise data in each capability dimension. Then, the electronic device may use a highest value of each capability dimension as a final score of the user in the capability dimension, or may use an average value of each piece of exercise data in the capability dimension as a final score of the user in the capability dimension, and determine, based on final scores in all capability dimensions, the athlete type corresponding to the user.

It should be noted that a step of determining the athlete type of the user may be completed locally on the electronic device, or may be completed by a cloud server. If the step of determining the athlete type of the user is completed by the cloud server, the electronic device may upload the obtained exercise data to the cloud server. The cloud server may determine the athlete type of the user based on the exercise data uploaded by the electronic device, namely, an identification result, and then feed back the identification result to the electronic device. After receiving the identification result fed back by the cloud server, the electronic device may determine the athlete type of the user, and perform subsequent steps.

Further, in another embodiment of this application, if the obtained exercise data includes a plurality of exercise items, S202 may be specifically implemented in the following two manners:
Manner 1: The athlete type of the user is determined by determining an optimal score corresponding to each exercise item.

In S2021, an optimal exercise score corresponding to the exercise item is determined based on the exercise data associated with each exercise item.

In this embodiment, the electronic device may divide all obtained exercise data into a plurality of data groups based on an exercise item to which the exercise data belongs, and each data group corresponds to a same exercise item. The electronic device may determine the optimal exercise score of the user for an exercise item based on a plurality of pieces of exercise data included in a data group associated with the exercise item. For example, the exercise data is exercise data of a running type. Different exercise items may be classified based on different running distances, for example, 5-kilometer running, 10-kilometer running, half-marathon running, and full-marathon running. The electronic device may identify a running distance in each piece of exercise data, to determine a data group to which the exercise data belongs. For example, if a running distance corresponding to a piece of exercise data is 5.5 kilometers, and the running distance is greater than "5-kilometer running" and less than "10-kilometer running", the exercise data may be identified as belonging to the exercise item "5-kilometer running". Certainly, the electronic device may alternatively intercept a data segment of the first 5 kilometers from the exercise data, and add the data segment to a data group corresponding to the exercise item "5-kilometer running".

In a possible implementation, a manner of determining the optimal exercise score may be: selecting a piece of exercise data with an optimal exercise score from the exercise item, and determining the optimal exercise score based on the selected exercise data with the best score.

Further, in another embodiment of this application, the manner of determining the optimal exercise score may further include the following steps:
Step 1: Determine an ideal heart rate corresponding to the exercise item, where the ideal heart rate is specifically a corresponding heart rate of a user in a full-effort exercise state.

In this embodiment, when performing exercise and training, the user does not necessarily perform exercise in a full-effort exercise state, that is, may reserve strength. Therefore, the collected exercise data cannot directly reflect the optimal exercise score of the user. In this case, the optimal exercise score of the user may be deduced based on the collected exercise data. The electronic device may determine, based on the heart rate of the user, an intensity coefficient corresponding to the current exercise. Therefore, a reference value, namely, the ideal heart rate, needs to be determined. The ideal heart rate is used to indicate a corresponding heart rate of the user in the full-effort exercise state, namely, a peak value that a heart rate reaches when the user exerts maximum effort to complete the exercise item.

If individual information of users is different, ideal heart rates corresponding to a same exercise item may also be different. For example, an ideal heart rate of a 20-year-old user may be higher than an ideal heart rate of a 30-year-old user, and an ideal heart rate of a male user may also be higher than an ideal heart rate of a female user of a same age group. In this case, the electronic device may obtain, based on personalized information of a user, an ideal heart rate associated with the individual information.

In a possible implementation, the electronic device may obtain a resting heart rate of the user. For example, the electronic device is a smartwatch. In addition to recording a dynamic heart rate of the user in an exercise state, the smartwatch may further obtain a heart rate of the user in a resting state, namely, the resting heart rate. Because there is usually a relative relationship between the ideal heart rate of the human body and the resting heart rate, the electronic device may determine, by using the resting heart rate of the user, the ideal heart rate corresponding to the user.

Step 2: Determine, based on an actual heart rate in the exercise data and the ideal heart rate, an intensity coefficient associated with the exercise data.

In this embodiment, the exercise data records an actual heart rate of the user in an exercise process, and the electronic device may determine, by calculating a ratio of the actual heart rate to the ideal heart rate, an intensity system corresponding to the current exercise. The ideal heart rate is a heart rate of the user in the full-effort exercise state. Therefore, if the actual heart rate of the user during exercise is closer to the ideal heart rate, an exercise status of the user is closer to the full-effort exercise state. It can be learned that the intensity coefficient corresponding to the user is determined by calculating a ratio of the actual heart rate to the ideal heart rate. Being close to the full-effort state indicates a corresponding high intensity coefficient.

Step 3: Determine, based on the intensity coefficient and an actual exercise score, the optimal exercise score corresponding to the exercise item.

In this embodiment, the electronic device may obtain, through calculation based on an intensity coefficient corresponding to the current exercise and an actual exercise score corresponding to the exercise data, a corresponding exercise score obtained when the user exercises in the full-effort state, namely, the optimal exercise score. For example, a manner of calculating the optimal exercise score may be: Optimal exercise score=Actual exercise score/Intensity coefficient=Actual exercise score/(Actual heart rate/Ideal heart rate).

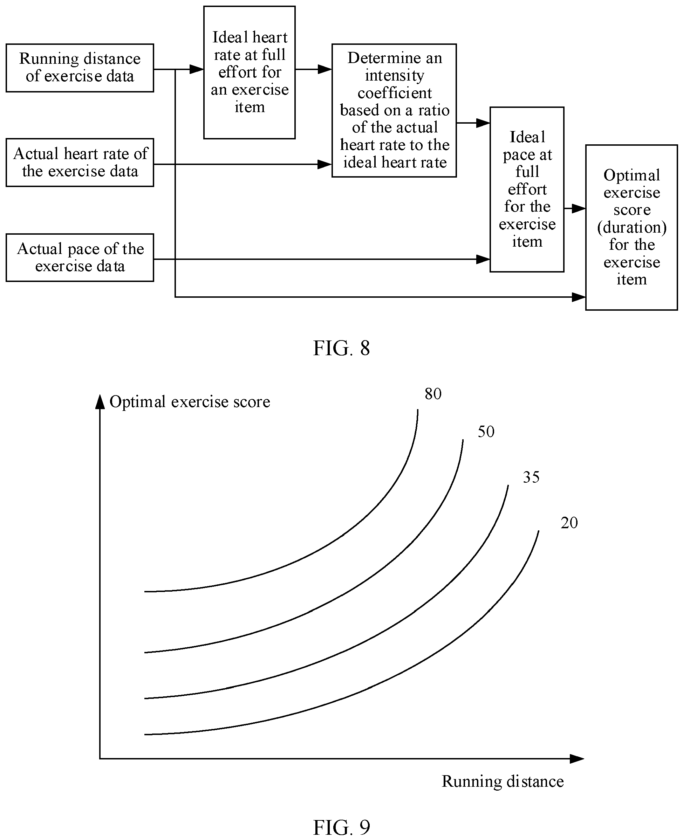

For example, running is used as an example to describe an optimal exercise score. FIG. 8 is a schematic diagram of calculating an optimal exercise score according to an embodiment of this application. Refer to FIG. 8. The electronic device may identify a running distance corresponding to exercise data, determine, based on the running distance, an exercise item to which the exercise data belongs, and determine, based on the exercise item, an ideal heart rate corresponding to the exercise item, namely, a heart rate value corresponding to the user when the user completes the running distance in a full-effort state. Then, the electronic device may determine, from the exercise data, an actual heart rate corresponding to the user in the current exercise process, calculate a ratio of the actual heart rate to an ideal heart rate, and determine an intensity coefficient corresponding to the current exercise data. For the running exercise, an exercise score is generally determined by using running duration, and may be converted by using an actual pace corresponding to the exercise data. In this case, the electronic device may calculate an ideal pace of the user in a full-effort exercise state based on the actual pace of the current exercise and the foregoing intensity coefficient, and calculate optimal running duration in an ideal state based on a ratio of a running distance to the ideal pace, and use the optimal running duration as the optimal exercise score corresponding to the exercise item.

In S2022, the optimal exercise score is imported into a normalization algorithm corresponding to the exercise item, and a normalized parameter corresponding to the exercise item is determined.

In this embodiment, exercise items corresponding to different exercise data may be different. For example, a time consumed for running 5 kilometers by a user is definitely shorter than a time consumed for running 10 kilometers. Therefore, a corresponding exercise score, namely, an exercise capability of the user, cannot be determined by using a same time indicator. On the other hand, exercise indicators in different exercise distances are not necessarily in a linear relationship. For example, a standard time consumed for a common user to run 5 kilometers is 30 minutes, and a standard time consumed for a common user to run 10 kilometers is not necessarily 30*2 minutes, namely, one hour, but may be one hour and 10 minutes. Therefore, for different exercise items, it is also impossible to directly compare sports scores by using a distance relationship between different items. Based on this, to better determine an exercise capability of the user, to determine an athlete type corresponding to the user, the electronic device may perform normalization processing on an optimal exercise score, to represent scores of different exercise items by using a unified dimension indicator, to improve accuracy of subsequently identifying the athlete type of the user.

In a possible implementation, the electronic device may store normalization algorithms corresponding to different exercise items. The electronic device may determine an exercise item to which exercise data belongs, and calculate, by using a normalization algorithm associated with the exercise item, a normalized parameter associated with the exercise item. The normalization algorithm may be specifically a corresponding relationship list, or may be a conversion function, and a form of the normalization algorithm is not limited herein.

In this embodiment, the electronic device may import the optimal exercise score obtained through calculation into a normalization algorithm of an exercise item corresponding to the optimal exercise score, to obtain a normalized parameter of the exercise item, so that optimal exercise scores of different exercise items can be converted into a same dimension for comparison.

For example, a running exercise is used as an example. FIG. 9 is a schematic diagram of a normalization algorithm according to an embodiment of this application. Refer to FIG. 9. A horizontal coordinate in a coordinate system is used to determine a running distance, and is used to distinguish different exercise items; and a vertical coordinate in the coordinate system is used to determine an optimal exercise score. Different parameter curves may be obtained based on different values of normalized parameters. For example, if a value of a normalized parameter is 20, the normalized parameter corresponds to curve 1 in FIG. 9; and if a value of a normalized parameter is 35, the normalized parameter corresponds to curve 2 in FIG. 9, that is, different curves correspond to different normalized values.

For example, the running exercise is still used as an example. Table 1 shows a correspondence table of normalized parameters provided in an embodiment of this application. Refer to Table 1. The electronic device may further determine, according to a preset correspondence table, a normalized parameter corresponding to each optimal exercise score. If an optimal exercise score corresponding to an exercise item that a user runs at 5 kilometers is 25 minutes, it may be determined, according to Table 1, that a normalized parameter of the user for running at 5 kilometers is 40. If an optimal exercise score corresponding to an exercise item of running at 10 kilometers of the user is 43 minutes, it may also be determined, according to Table 1, that a normalized parameter of the user for running at 10 kilometers is 50. For another exercise item, a normalized parameter corresponding to the another exercise item may also be obtained by querying the correspondence table with reference to the foregoing process.

**Table 1**

| Normalized parameter | 5-kilometer optimal exercise score | 10-kilometer exercise score | 21-kilometer optimal exercise score | 42-kilometer optimal exercise score |
|---|---|---|---|---|
| ... | ... | ... | ... | ... |
| 30 | 32 minutes | 1 hour and 5 minutes | 2 hours and 25 minutes | 5 hours and 15 minutes |
| 40 | 25 minutes | 51 minutes | 1 hour and 53 minutes | 3 hours and 55 minutes |
| 50 | 21 minutes | 43 minutes | 1 hour and 38 minutes | 3 hours and 20 minutes |
| ... | ... | ... | ... | ... |

In S2023, the athlete type of the user is determined based on a normalized parameter of each exercise item.

In this embodiment, because different exercise items may reflect exercise capabilities corresponding to the user in different exercise evaluation dimensions, for example, an exercise item may mainly reflect explosive power of the user, and another exercise item mainly reflects endurance of the user, a comprehensive assessment can be made on the exercise capabilities of the user by using normalized parameters of different exercise items. Therefore, the athletic type of the user may be determined by using normalized parameters corresponding to different exercise items.

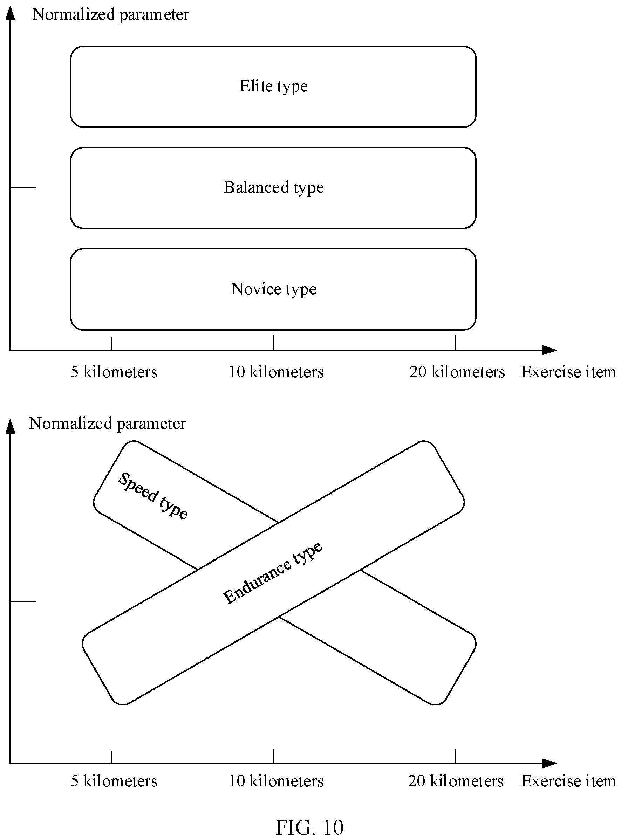

For example, a running exercise is used as an example for description. FIG. 10 is a diagram of a correspondence between athlete types according to an embodiment of this application. Refer to FIG. 10. Different athlete types correspond to different areas in a coordinate system. A corresponding athletic curve is generated in the coordinate system based on normalized parameters corresponding to different exercise items of a user, and an athlete type corresponding to the user is determined based on an area in which the athletic curve falls. It may be determined by using FIG. 10 that, the athlete type may be classified into: a novice type (that is, a normalized parameter corresponding to each exercise item is at a low level), a balanced type (that is, a normalized parameter corresponding to each exercise item is at a medium level), an elite type (that is, a normalized parameter corresponding to each exercise item is at a high level), an endurance type (a normalized parameter corresponding to a short-distance exercise item is low, and a normalized parameter corresponding to a long-distance exercise item is high), and a speed type (a normalized parameter corresponding to a short-distance exercise item is high, and a normalized parameter corresponding to a long-distance exercise item is low).

In this embodiment of this application, the optimal exercise score corresponding to each exercise item is determined based on all exercise data, then normalization processing is performed on optimal exercise scores of different exercise items to obtain a normalized parameter of each exercise item, and the athlete type of the user is determined based on the normalized parameters of all exercise items, so that accuracy of a process of identifying the athlete type to which the user belongs can be improved.

### Manner 2: The athlete type of the user is determined based on normalized parameters corresponding to all exercise data

In S2021', a normalization algorithm associated with the exercise data is determined based on an exercise item corresponding to the exercise data, and the normalized parameters corresponding to the exercise data is determined by using the normalization algorithm.

In this embodiment, similar to the foregoing implementation, different exercise items may correspond to different normalization algorithms, so that exercise data corresponding to each exercise item can be uniformly compared in a same dimension. Based on this, the electronic device may identify an exercise item of the exercise data, obtain a normalization algorithm corresponding to the exercise item, and calculate, by using the normalization algorithm corresponding to the exercise item, a normalized parameter corresponding to the exercise data. For an implementation process of calculating the normalized parameter, refer to the description of the foregoing manner. For example, the normalized parameter is calculated by using a correspondence table or a related conversion function. Details are not described herein again.

In S2022', the athlete type of the user is determined based on the normalized parameters corresponding to all exercise data.

In this embodiment, the electronic device may obtain a normalized parameter corresponding to each piece of exercise data obtained, and determine a performance capability of the user in the exercise item by using normalized parameters of a plurality of pieces of exercise data in the exercise item. For example, an average value of normalized parameters of all exercise data in an exercise item may be used as a feature value corresponding to the user in the exercise item, and then the athlete type of the user is determined based on feature values corresponding to the user in all exercise items. Similar to the foregoing manner, different exercise items may reflect different exercise capabilities of the user, such as endurance and explosive power. Therefore, a feature value of a corresponding exercise item is determined by using a plurality of pieces of exercise data, so that a performance capability of the user for the exercise item in a plurality of exercises can be determined, that is, stability of evaluation of a performance capability of a specific exercise item is improved, and accuracy of athletic type identification is improved.

In this embodiment of this application, the electronic device does not determine, from the optimal exercise score, the normalized parameter corresponding to the exercise item, but performs normalization processing on all exercise data, so that a performance capability of the user in a particular exercise item can be determined from a plurality of exercises, thereby improving stability of evaluating a performance capability of the exercise item.

In S203, a training direction of the user is determined based on the athlete type, and a training plan matching the training direction is generated.

In this embodiment, after determining the athlete type of the user, the electronic device may determine the training direction of the user. Because the athlete type may determine exercise capabilities of the user in different dimensions, running is used as an example for description, if the athlete type of the user is a sprint type, it indicates that the user has a good explosive power but a poor endurance. In this case, a subsequent training direction may be to improve the endurance of the user and maintain the explosive power of the user. If the athlete type of the user is a novice type, it indicates that the user has a poor explosive power, poor endurance, and the like. In this case, a subsequent training direction is to improve the explosive power and endurance of the user. It can be learned that a direction in which the user is good at and a direction to be promoted may be determined by using the athlete type, so that a corresponding training direction obtained when the user is trained, or referred to as a training focus is determined.

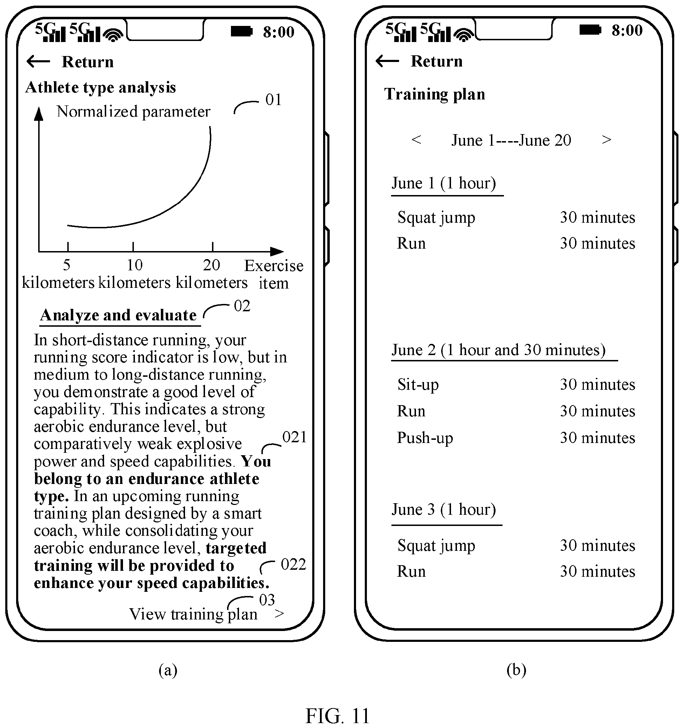

In a possible implementation, after generating the training plan, the electronic device may display a training report corresponding to the user, where the training report includes the training plan and a descriptive paragraph about the athletic type and the training direction. The training report specifically includes two parts: an analysis page for evaluating an overall exercise capability of the user, and a page for viewing the training plan. The analysis page generated by the electronic device may include a description paragraph of an athlete type and a training direction of the user, so that the user can determine an exercise capability of the user by using the analysis page. For example, FIG. 11 is a schematic diagram of a training report according to an embodiment of this application. Refer to (a) in FIG. 11. After obtaining exercise data of a user, that is, after obtaining the exercise data of the user in FIG. 5(f), the electronic device may determine an athlete type and a training direction of the user, and then generate an analysis page in (a) in FIG. 11. The analysis page displays normalized parameters that are of different exercise items and that are determined based on the collected exercise data, that is, a chart area 01 in (a) in FIG. 11. Then, an analysis paragraph 02 used to describe the athlete type and the training direction of the user may be further generated. The user may determine an athlete type 021 and a training direction 022 of the user by using the analysis paragraph.

In this embodiment, after determining the training direction of the user, the electronic device may extract, from a preset training course library, a training course that matches the training direction, to generate a training plan that matches the training direction.

In a possible implementation, the training plan may include a training period, a training time, and a training course corresponding to the training time. For example, refer to (a) in FIG. 11. The analysis page further includes a "view a training plan" control 03. If the electronic device detects that the user taps the control 03, the electronic device may display a training plan corresponding to the user. As shown in (b) in FIG. 11, the training period is specifically used to indicate duration of the training plan, for example, June 1 to June 20. The training time specifically indicates a trigger time and course duration of each training course. For example, training is performed for one hour on June 1. The training course specifically indicates training content. For example, training content corresponding to June 1 is deep squatting and running.

It should be noted that the foregoing operation of determining the training plan may be locally completed on the electronic device, or may be completed on a cloud server. If the operation of determining a training plan is completed on a cloud server, the training course library may be stored on the cloud server. The cloud server determines a training direction of the user based on the athlete type of the user, extracts, from the training course library, a training course that matches the training direction, generates a training plan corresponding to the training course, and feeds back the training plan to the electronic device. When feeding back the training plan to the electronic device, the cloud server may send a complete training plan of the user to the electronic device. Alternatively, the cloud server may feed back a training course in the training plan to the electronic device when a training prompt moment is reached. For example, the cloud server sends a training course corresponding to a current day to the electronic device. Alternatively, the cloud server may receive a plan query request initiated by the user and sent by the electronic device, and send a training course corresponding to the plan query request in the training plan to the electronic device.

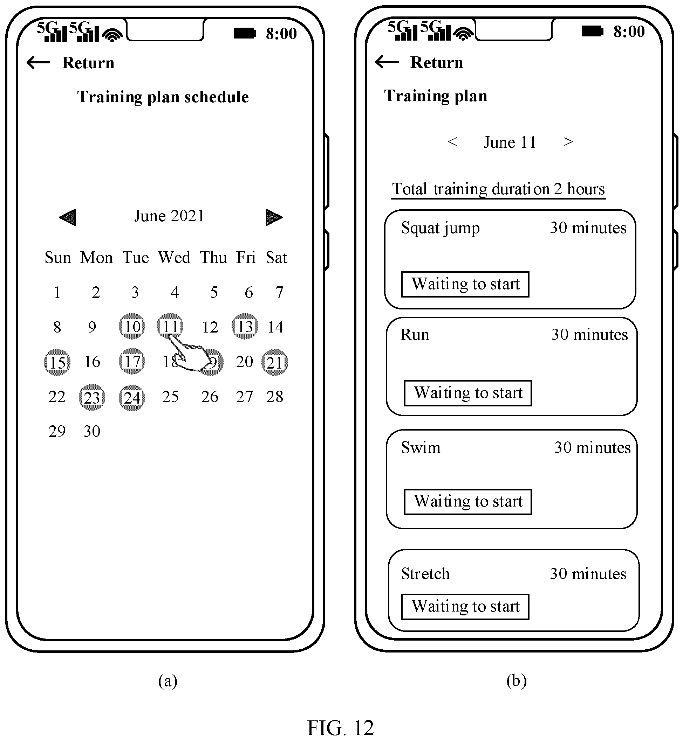

For example, FIG. 12 is a schematic diagram of querying a training plan according to an embodiment of this application. Refer to (a) in FIG. 12. A training calendar may be displayed on the electronic device, and a date having a training course in the training calendar may be marked in a preset manner. In the figure, June 10 and 11 are dates having a training course, and June 30 is a date having no training course. When a user needs to query a training course of a specific date in a training plan, the user can tap a control of the corresponding date in the training calendar to initiate a plan query request. If the electronic device locally stores the training course corresponding to the date, the electronic device may display the training course corresponding to the date. If the electronic device locally does not store the training course corresponding to the date, the electronic device may send the plan query request to the cloud server. The cloud server feeds back the training course corresponding to the date to the electronic device based on the training plan corresponding to the user, and the electronic device displays the training course corresponding to the date, as shown in (b) in FIG. 12.

In a possible implementation, if the training purpose of the user is obtained, S203 is specifically: determining the training direction of the user based on the training purpose and the athlete type. The electronic device may determine, by presetting a training purpose by the user, a capability that needs to be improved, and determine a current exercise capability of the user based on the identified athlete type. Therefore, the electronic device determines a corresponding training direction by identifying whether a capability required for the training purpose matches the current exercise capability, and generates a training plan corresponding to the determined training direction.

The running exercise is used as an example for description. If the training purpose of the user is "participating in a full marathon race", and the identified athlete type of the user is a sprint type, in this case, it indicates that the user has an excellent explosive power but poor endurance. Based on the foregoing training purpose, a required capability is excellent endurance, and current endurance of the user does not match the training purpose. In this case, it may be determined that the training direction is to improve endurance, and a training plan for improving endurance is generated. It can be learned from the foregoing that, according to the generation method for a training plan provided in this embodiment of this application, exercise data of a user in a historical exercise process may be obtained, an athlete type of the user may be determined, a training direction corresponding to training performed by the user may be identified based on the athlete type, and a training plan that matches the training direction is generated, so that a training plan that adapts to an exercise capability of the user can be generated. Because an individual parameter of the user cannot directly reflect the exercise ability of the user, the training plan generated based on the individual parameter may not match the exercise ability of the user. However, the exercise data that is collected and that is in a historical exercise activity of the user can reflect an actual exercise ability of the user. The athlete type of the user is determined based on performance of the user in historical exercise, so that a matching degree between the training plan and the actual exercise ability of the user can be improved. In this way, the generated training plan can be used to guide the user to perform exercise and training more pertinently, and accuracy of the generated training plan is improved.

### Embodiment 2

In Embodiment 1, an athlete type of a user is mainly determined based on exercise data, a corresponding training direction is determined based on the athlete type, and then a training plan that matches the training direction is generated. In this embodiment, an implementation process of how to generate a training plan based on a training direction is specifically described. Compared with the foregoing embodiment, in this embodiment, a training direction of a user obtained by an electronic device may be obtained in the manner in Embodiment 1, or may be obtained in a manner preset by the user, or may be obtained in a manner of receiving feedback from another device. A manner of obtaining the training direction is not limited herein.

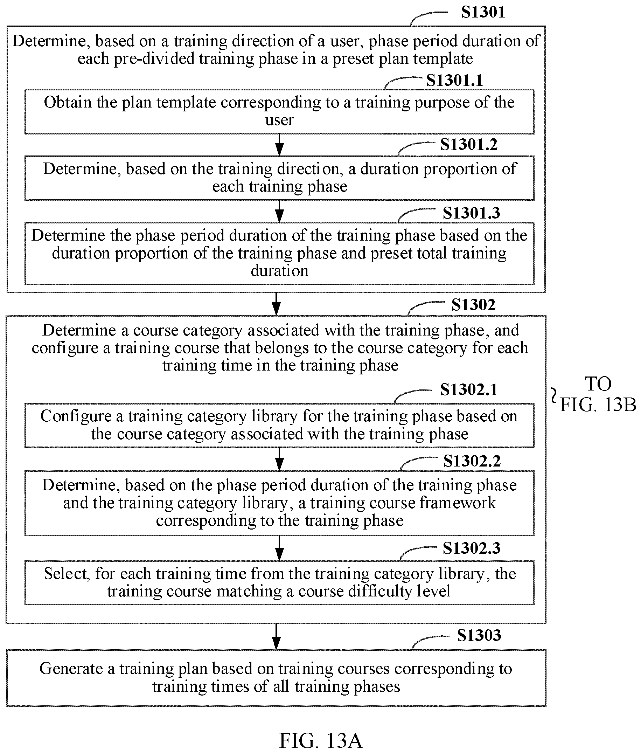

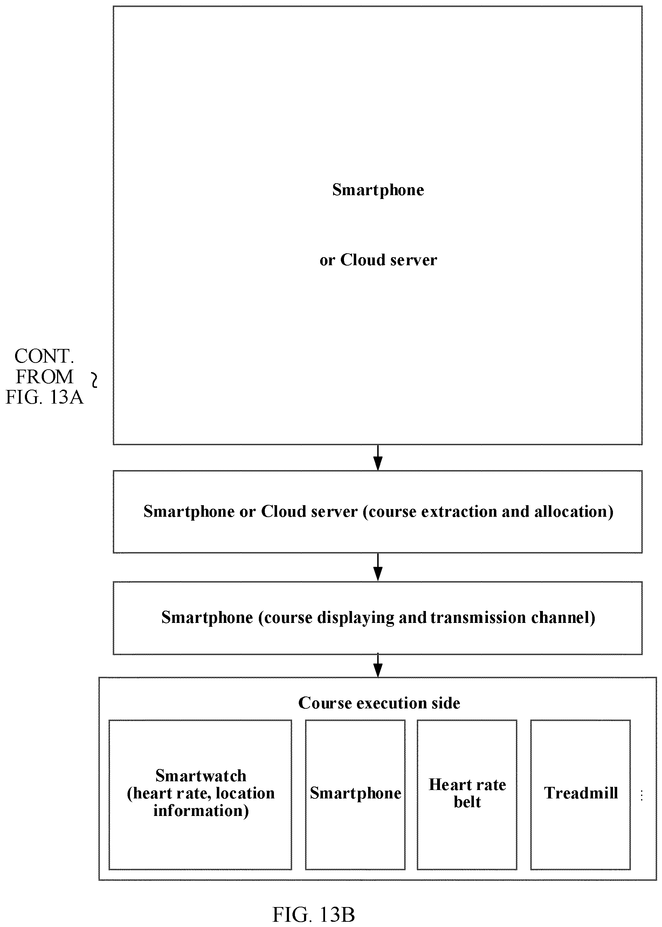

In this embodiment of this application, a procedure is executed by an electronic device. The electronic device includes but is not limited to a device that can execute a training plan generation task, such as a computer, a smartphone, a notebook computer, a tablet computer, a wearable intelligent device (such as a smart necklace, a smart watch, and a smart band), and a cloud server. FIG. 13A and FIG. 13B are an implementation flowchart of a generation method for a training plan according to another embodiment of this application. Details are as follows:
In S1301, phase period duration of each pre-divided training phase in a preset plan template is determined based on a training direction of a user.

As described above, the training direction in this embodiment may be determined in the manner in Embodiment 1. Optionally, the electronic device may further generate a setting interface of the training direction, and the user may fill in the training direction for the training plan in the setting interface, so that the electronic device can also determine the training direction in a user setting manner. Optionally, if the user has identified the training direction of the user on another device, in this case, the electronic device may receive the training direction of the user fed back by the another device. If the electronic device is a user terminal, and the another device is a cloud server, the electronic device may locally install a client program corresponding to the cloud server, and log in to an account associated with the user in the client program. If the cloud server stores a training direction that has been identified by the user, the cloud server may deliver the training direction to each device that has logged in to the account associated with the user, namely, the electronic device provided in this embodiment, so that the electronic device can obtain the training direction of the user.

In this embodiment, the electronic device may prestore a plan template corresponding to the training plan. The plan template is used to limit a plurality of different training phases of the user during training. Each training phase has corresponding phase period duration, that is, indicates duration of the training phase. Different training phases correspond to different training focuses. Based on this, after determining the training direction of the user, the electronic device may correspondingly adjust the phase period duration of each training phase in the training plan, so that the training period duration of each training phase matches the training direction. Specifically, different training phases may be associated with one or more training focuses. If a training focus of a training phase matches the training direction of the user, phase period duration of the training phase may be prolonged. Otherwise, if a training focus of the training phase does not match the training direction of the user, phase period duration of the training phase may be shortened.

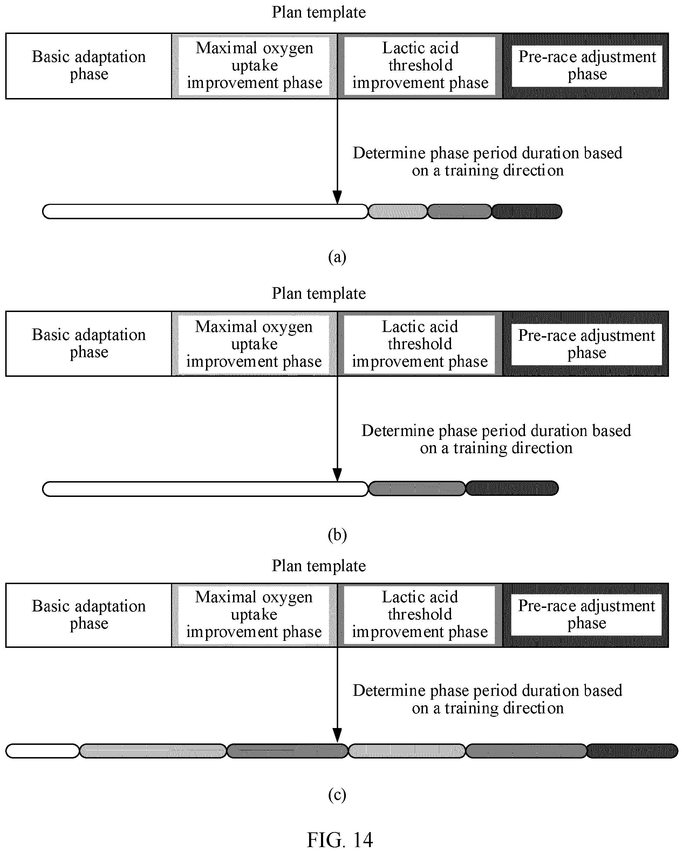

For example, a running exercise is used as an example for description. FIG. 14 is a schematic diagram of division of training phases according to an embodiment of this application. Refer to (a) in FIG. 14. A plan template includes four training phases: a basic adaptation phase, a maximal oxygen uptake improvement phase, a lactic acid threshold improvement phase, and a pre-race adjustment phase. In the plan template, phase period duration corresponding to each training phase is not limited. The basic adaptation phase is specifically to enable the user to gradually enter a continuous exercise training state. Therefore, a training focus of this phase is balanced improvement in all directions. The maximal oxygen uptake improvement phase is mainly used to increase maximal oxygen uptake of the user, and the maximal oxygen uptake is related to a running pace of the user, that is, a training focus of this phase is to increase the running pace of the user. The lactic acid threshold improvement phase is mainly used to improve a user capability of lactic acid metabolism. Lactic acid is generated during a long-distance running process, and lactic acid accumulation occurs after the long-distance running. That is, a training focus of this phase is to improve endurance of the user. The pre-race adjustment phase is specifically to keep physical strength of the user and ensure a specific amount of exercise. That is, a training focus of this phase is to maintain a capability. It can be learned that different training phases correspond to different training focuses. If the athlete type of the user is a novice type, a corresponding training direction is balanced improvement. It may be determined by using training focuses of the foregoing phases that the training direction of the user matches the basic adaptation phase. In this case, phase period duration corresponding to the phase may be prolonged. As shown in (a) in FIG. 14, phase period duration of the basic adaptation phase is longer than phase period duration of the other three phases, phase period duration of another phase may also be configured as a matching value based on the training direction.

In a possible implementation, phase period duration of a training phase in the plan template may be 0, that is, a subsequently generated training plan may not include some training phases in the plan template. As shown in (b) in FIG. 14, after phase period duration of each training phase is determined based on the training direction, the phase period duration allocated to the maximal oxygen uptake improvement phase is 0, that is, the phase is not included, and only the other three phases are left. There are two possibilities for the foregoing case. In a first case, the user has good performance on a training focus corresponding to the training phase, and therefore, a corresponding exercise capability does not need to be improved through the training phase. For example, the user has extremely good endurance performance, and belongs to a very excellent level, but has a low speed. In this case, a lactic acid threshold improvement phase may not be performed, but a maximal oxygen uptake improvement phase may be increased. In another case, total training duration of the user is short. In this case, proper training period duration cannot be allocated to all training phases. Therefore, some training phases may be deleted, that is, training period duration of a training phase is set to 0.

In a possible implementation, the training phases may be periodically arranged in the plan template. As shown in (c) in FIG. 14, the maximal oxygen uptake improvement phase and the lactic acid threshold improvement phase in the plan template are alternately arranged, and appear in the plan template for a plurality of times. In this case, the electronic device may determine, based on the training direction of the user, phase period duration corresponding to each training phase in each period. Certainly, the electronic device may also determine total duration of the training phase, and configure corresponding phase period duration for each training period of the training phase based on a quantity of times that the training phase appears in the plan template. As shown in (c) in FIG. 14, total duration of the maximal oxygen uptake improvement phase is 28 days, and there are two training periods in the plan template. The electronic device may allocate 13 days to the first training period of the maximal oxygen uptake improvement phase, and allocate 15 days for the second training cycle of the maximal oxygen uptake improvement phase.

Further, in another embodiment of this application, S1301 may specifically include the following types:
In S1301.1, the plan template corresponding to the training purpose of the user is obtained.

In this embodiment, the electronic device may store different plan templates, and the different plan templates are associated with corresponding training purposes. Based on this, before generating the training plan of the user, the electronic device may obtain the training purpose of the user. A manner of obtaining the training purpose is described above, and an information input interface shown in FIG. 4(a) to FIG. 4(c) is generated, to prompt the user to enter the individual parameter and the training purpose of the user. After obtaining the training purpose of the user, the electronic device selects a plan template that matches the training purpose from the plan template library.

It should be noted that, quantities of training phases and training focuses included in plan templates corresponding to different training purposes may be different. For example, if a training purpose of a user A is to improve physical quality, a corresponding plan template may be a template 1, and the template 1 may include three training phases: a physical energy recovery phase, a physical energy improvement phase, and a physical energy stability phase. A training purpose of a user B is to participate in a marathon race, and a corresponding plan template may be a template 2. The template 2 may include four training phases: a basic adaptation phase, a maximal oxygen uptake improvement phase, a lactic acid threshold improvement phase, and a pre-race adjustment phase. Therefore, a training phase included in a plan template and a training focus corresponding to the training phase match a training purpose corresponding to the training phase, so that a degree of adaptation between a generated training plan and a user can be improved.

In S1301.2, a duration proportion of each training phase is determined based on the training direction.

In this embodiment, an initial proportion may be preconfigured for a duration proportion of training phases in the plan template. For example, a ratio of different training phases is 1:1, that is, duration of each training phase is consistent. After determining the training direction (namely, a training focus) of the user, the electronic device may adjust an initial ratio between training phases in the plan template. An adjustment basis is based on whether a training focus corresponding to a training phase matches the training direction of the user. If the training focus corresponding to a training phase matches the training direction of the user, an initial proportion corresponding to the training phases may be increased to obtain the foregoing duration proportion. If the training focus corresponding to a training phase does not match the training direction of the user, the initial proportion of the training phase may be shortened to obtain a corresponding duration proportion, or the initial proportion may remain unchanged, that is, the initial proportion of the training phase is used as a duration ratio.

In S1301.3, the phase period duration of the training phase is determined based on the duration proportion of the training phase and preset total training duration.

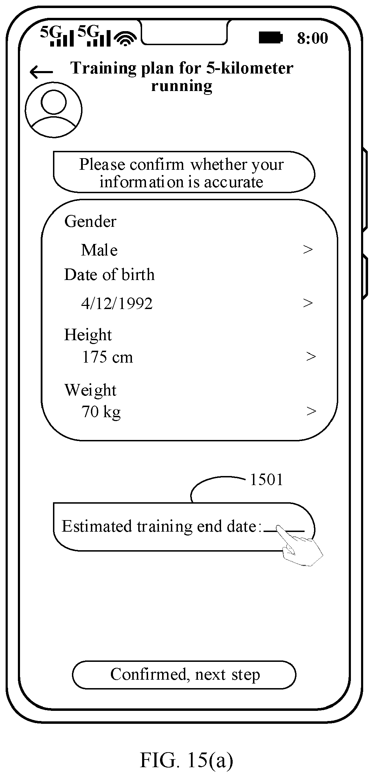

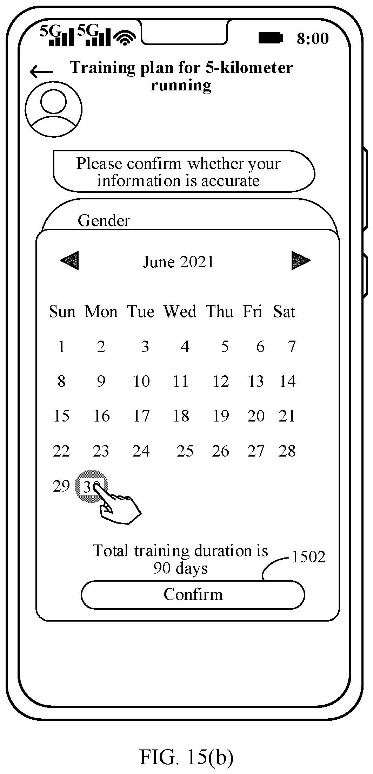

In this embodiment, when the user needs to generate the training plan, the user may enter the total training duration of the current training plan on the electronic device. For example, on the information input interface shown in FIG. 4(a) to FIG. 4(c), in addition to the individual parameter and the training purpose that are entered by the user, the user may further enter a corresponding total training duration, for example, "90 days" in FIG. 4(a) to FIG. 4(c). When setting the total training duration, the user may further determine the total training duration by tapping a corresponding training end date. For example, FIG. 15(a) to FIG. 15(c) are schematic diagrams of setting total training duration according to an embodiment of this application. Refer to FIG. 15(a). An electronic device may generate an information input interface. Similar to FIG. 4(a) to FIG. 4(c), the information input interface may be used to input a personal parameter and a training purpose of a user. In addition, a control 1501 used to fill in an "estimated training end date" is further configured. When detecting that the user taps the control 1501, the electronic device generates a training calendar. As shown in FIG. 15(b), the electronic device may determine a training end date selected by the user. For example, June 30, based on a selection operation of the user, the electronic device determines total training duration based on a difference between a current date and the training end date, and displays the total training duration in the training calendar, to prompt the user of the total training duration of a current training plan; and if it is detected that the user taps a confirmation control 1502, uses a date selected by the user as an estimated training end date, to determine the total training duration.

In a possible implementation, a start date of the training plan is optional. The electronic device may use a corresponding current date on which the training plan is generated as an optional date of the training plan, or may set a date based on a requirement of the user. As shown in FIG. 15(b), the user may select a start date and an end date in the training calendar.

In this embodiment, the electronic device may obtain the phase period duration of each training phase by calculating a product of a duration proportion corresponding to each training phase and the total training duration corresponding to the user. For example, if a duration proportion of a training phase is 25%, and total training duration is 60 days, phase period duration corresponding to the training phase is 25%*60 days=15 days. Similarly, another training period may also be calculated in the foregoing manner.

In a possible implementation, if the duration ratio is a ratio of training phases, for example, a plan template includes four training phases, a duration ratio of the four training phases is: phase 1 :phase 2:phase 3:phase 4=1:1:2:2, in this case, it may be determined that the phase 1 or the phase 2 accounts for one sixth of total training duration, and the phase 3 or the phase 4 accounts for one third of the total training duration. Then, the phase period duration of the training phase may be obtained through calculation by calculating the total training duration and a proportion of the training phase.

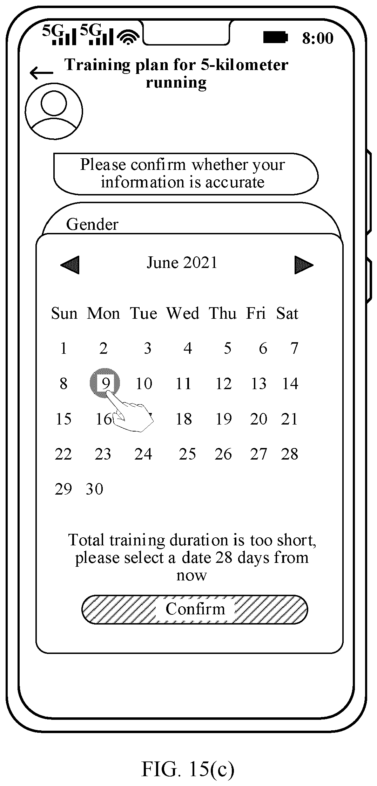

In a possible implementation, a duration range of the total training duration is from 28 days to 180 days, that is, shortest total training duration of the training plan is one month, and longest total training duration is six months. Because the total training duration is excessively short, when the user cultivates an exercise habit, the training is close to the end, and improvement in the training direction of the user cannot be effectively performed. On the other hand, if the total training duration of the user is excessively long, a change degree of an exercise capability of the user is large, and determining the training direction and a proportion of a training phase based on an initial state may be no longer applicable to the user. Therefore, accuracy of the training plan is greatly reduced. Therefore, to consider both accuracy of the training plan and execution feasibility, the electronic device may set a corresponding effective range for the total training duration. If the total training duration set by the user is beyond the foregoing effective duration range, corresponding prompt information may be generated, as shown in FIG. 15(c), the user is prompted that the total training duration is too short, and a date 28 days from now needs to be selected.

In this embodiment of this application, the training purpose of the user is identified, the plan template matching the training purpose is obtained, and the phase period duration of each training phase is determined based on the total training duration, so that accuracy of a period division process can be improved, and a degree of adaptation between a subsequent training plan and the user can be improved.

In S1302, a course category associated with the training phase is determined, and a training course that belongs to the course category is configured for each training time in the training phase, where the training time is determined based on the phase period duration of the training phase.

In this embodiment, the electronic device may divide the training time into a plurality of training times based on the phase period duration corresponding to the training phase. For example, if the training time is divided in a unit of day, each training time corresponds to one training date; or if the training time is divided in a unit of hour, each training time corresponds to one training hour. Certainly, different training dates may be consecutive or inconsecutive, which is specifically set based on an actual situation. If the electronic device is a user terminal on a user side, S1302 may be completed by using an application installed on the user terminal. If the electronic device is a cloud server, S1302 may further include an operation of determining a training course of each training time on the cloud server.

It should be noted that one training phase may correspond to one course category, or may correspond to more than two course categories, which is specifically determined based on an actual situation.

In this embodiment, the electronic device may pre-store a plurality of different training courses, and each training course may correspond to one course category. Because different training phases correspond to different training focuses, the electronic device may determine, based on whether the course category matches the training focus of the training phase, whether the course category is a course category associated with the training phase, and associate the training course associated with the course category with each training time. One training time may correspond to one or more training courses, and training courses in different training times may be the same or may be different.

In a possible implementation, the electronic device may record a correspondence table between a course category and a training phase, and may determine, by querying the correspondence table, a course category associated with each training phase.

In a possible implementation, S1302 may specifically include the following steps:
In S1302.1, a training category library is configured for the training phase based on the course category associated with the training phase.

In this embodiment, after determining course categories corresponding to a training phase, the electronic device may add all course categories corresponding to the training phase to a same training category library. The training category library includes at least one course category related to the training phase, and one course category may include candidate courses of different course difficulty levels.

A running exercise is used as an example for description. If a training phase is a maximal oxygen uptake improvement phase, the training category library may include three course categories: a high intensity interval type, an easy running type, and an aerobic endurance running type. Based on the foregoing course categories, candidate courses of different course difficulty levels are included. For example, for the aerobic endurance running type, a candidate course of low difficulty has a short distance and a low required running pace, and a candidate course of high difficulty has a long distance and a high required running pace. The electronic device may add candidate courses of different course difficulty levels in all course categories related to the training phase to a same training category library, to determine a training course corresponding to each training time in a subsequent step.

In S1302.2, a training course framework corresponding to the training phase is determined based on the phase period duration of the training phase and the training category library, where the training course framework is used to determine the training time included in the training phase and a course difficulty level corresponding to each training time.

In this embodiment, the electronic device may divide phase period duration to obtain a plurality of training times. As described above, the phase period duration may be divided into a plurality of training dates by using a date as a unit, and each training date is a training time. The training time may include a training date and training duration corresponding to the training date.

In this embodiment, the electronic device may configure corresponding course difficulty levels for different training dates, to generate a training course framework that meets a preset exercise intensity combination. For example, exercise intensity in an early stage of a training phase is low, and therefore a corresponding course difficulty level is low; and a user in a later stage of the training phase has adapted to exercise and training, corresponding exercise intensity is high, and therefore a corresponding course difficulty level is high. Different training phases may correspond to different exercise intensity combinations. For example, for a maximal oxygen uptake improvement phase, overall exercise intensity of the phase is higher than exercise intensity of a pre-race adjustment phase. Therefore, corresponding exercise intensity combinations are also different, that is, different training times have different course difficulty levels. By configuring the training course framework, the electronic device can implement refined configuration on an entire training phase, and limit a combination of a structure, duration, and difficulty of a training course included in the training phase, so that a relationship between course difficulty levels that need to be configured in different training times can be considered, and course difficulty levels of the entire training phase are not configured to be of a same value. This improves rationality and accuracy of training course selection, and further improves accuracy of a training plan for user training guidance.

In a possible implementation, the electronic device may obtain an athlete type of the user, and determine, based on the athlete type of the user, a course difficulty level corresponding to each course category.

In a possible implementation, the training course framework is further used to determine a course category corresponding to each training time.

In S1302.3, the training course matching the course difficulty level is selected for each training time from the training category library.

In this embodiment, after generating the training course framework corresponding to the training phase, the electronic device may configure a corresponding training course for each training time in the training course framework. Because each training time is associated with a corresponding course difficulty level, the electronic device may extract, from the training category library, a candidate course corresponding to the course difficulty level as a training course of the training time.

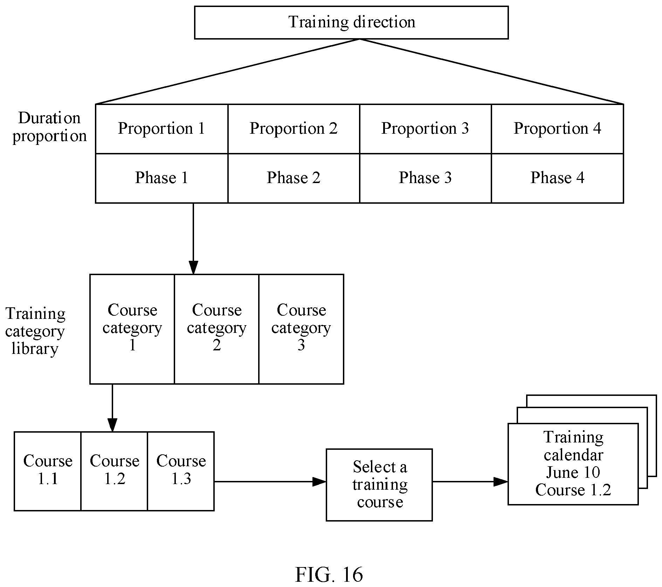

For example, FIG. 16 is a schematic diagram of selecting a training course according to an embodiment of this application. Refer to FIG. 16. An electronic device determines phase period duration of each training phase based on a training direction, and a corresponding training category library is configured for each phase period duration. For example, a training category library of a phase 1 includes three course categories, and each course category includes candidate courses of different course difficulty levels, namely, a course 1.1 to a course 1.3. The electronic device selects, from the course category based on a course difficulty level associated with a training time in the training phase, a candidate course of a corresponding course difficulty level as a training course of the training period, for example, if a course difficulty level corresponding to a training time on June 10 is 2, a candidate course whose course difficulty level is 2 is selected from a course category 1 as a training course corresponding to the candidate course, namely, the course 1.2.

In this embodiment of this application, a corresponding training category library and a corresponding training course framework are configured for each training phase, so that a training course corresponding to each training time in the training phase is determined, and association between training courses configured in different training times can be considered, thereby improving accuracy of a training plan.

In S1303, the training plan is generated based on training courses corresponding to training times of all training phases.

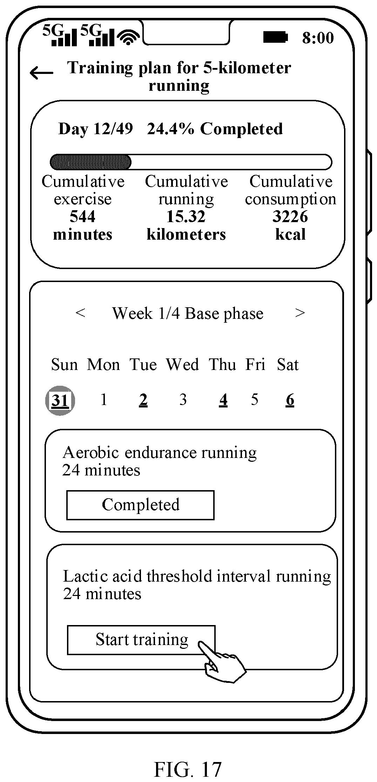

In this embodiment, the electronic device may integrate the training courses of the training times in each training phase, to obtain the training plan of the user. The electronic device may display, on a same interface, the training courses corresponding to all training times, as shown in (b) in FIG. 11, or may generate a training calendar interface based on a course corresponding to each training time, as shown in FIG. 17. FIG. 17 is a schematic diagram of a training calendar interface according to an embodiment of this application. Refer to FIG. 17. The training calendar interface displays a plan name of the training plan, for example, "training plan for 5-kilometer running". For a sequence number corresponding to a current training time, when a training time is divided in a unit of day, the sequence number is a number of days of training, for example, the 12^{th} day shown in the figure. Total training duration, that is, the foregoing 49 days, and a completion proportion of the current training plan, namely, 5.1%, may be further displayed, and the training calendar interface may further collect statistics on the training duration, an exercise distance, and accumulated consumption of the user, to obtain a corresponding value. The training calendar page further displays a training phase corresponding to a current training time, for example, a "base phase" in the figure that indicates a training phase corresponding to the training time, and may further display a phase sequence number corresponding to the training phase and a total quantity of training phases. The training calendar page may further display a date corresponding to the training time, that is, the 31^{st} day, and a date (namely, a training time) of a training course is marked in a preset manner, for example, the 31^{st} day, the 2^{nd} day, the 4^{th} day, and the 6^{th} day. The training time includes two training courses: "aerobic endurance running" and "lactic acid threshold interval running", and a training time and a completion status of a corresponding training course are displayed. For a completed training course, a control may be changed to "completed", and for a to-be-completed training course, a control may be displayed as "start training", so that the user can perform the training course.

In this embodiment, after the training plan of the user is determined, if the electronic device is a smartphone on a user side, the electronic device may display a training course of each training time in the training plan by using an application installed on the smartphone; or if the electronic device is a cloud server, the cloud server may send a training course corresponding to each training time to a user terminal corresponding to the user, and display the training course by using the smartphone. Certainly, if the user completes training by using a corresponding execution terminal in a training process, for example, needs to use a treadmill, a smartwatch, or a smart band, a received training course may be forwarded to each execution terminal by using a smartphone, so that the execution terminal collects exercise data of the user in a training process.

It can be learned from the foregoing that, in the generation method for a training plan provided in this embodiment of this application, phase period duration of each training phase in a training plan may be adjusted based on a training direction of a user. Different training phases correspond to different training focuses. Therefore, a corresponding course category may be associated, and a corresponding training course is configured for each training time based on a course category corresponding to the training phase, to generate a training plan that matches the training direction of the user. In this way, the training plan can improve a capability of the user in the training direction in a targeted manner, and the training plan is not determined based on an individual parameter of the user. This improves adaptation between the training plan and the user and improves pertinence of a training process.

### Embodiment 3

Embodiment 1 and Embodiment 2 mainly describe how to generate a training plan of a user. In this embodiment, how to adjust the training plan after the training plan of the user is generated is specifically described. Compared with the foregoing two embodiments, the training plan that needs to be adjusted in this embodiment may be generated in the manner in Embodiment 1, or may be generated in the manner in Embodiment 2, or may be generated in a manner obtained by combining Embodiment 1 and Embodiment 2 (that is, the training direction in Embodiment 2 is determined based on the manner in Embodiment 1, and then the training plan of the user is generated in the manner in Embodiment 2), or may be generated in another manner. A manner of generating the to-be-adjusted training plan is not limited herein.

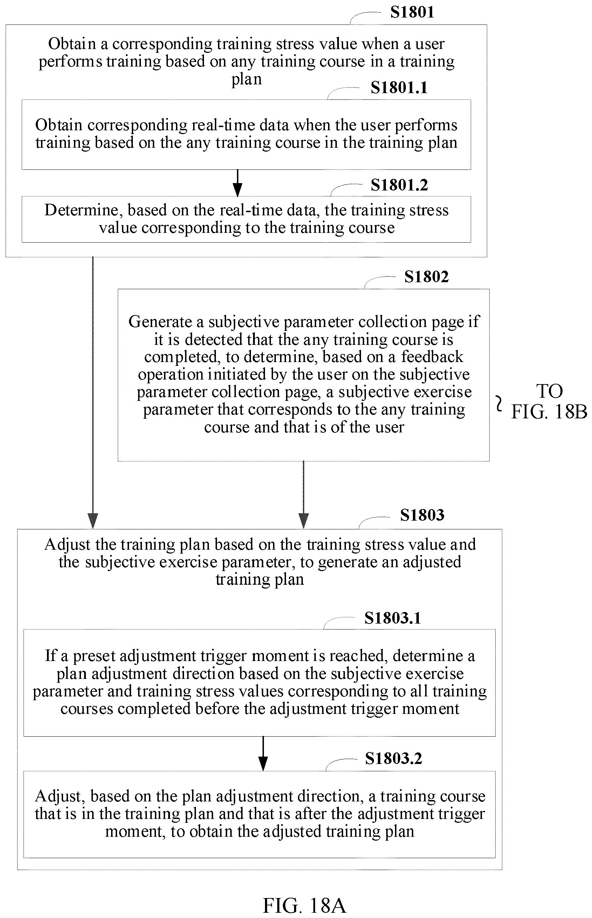

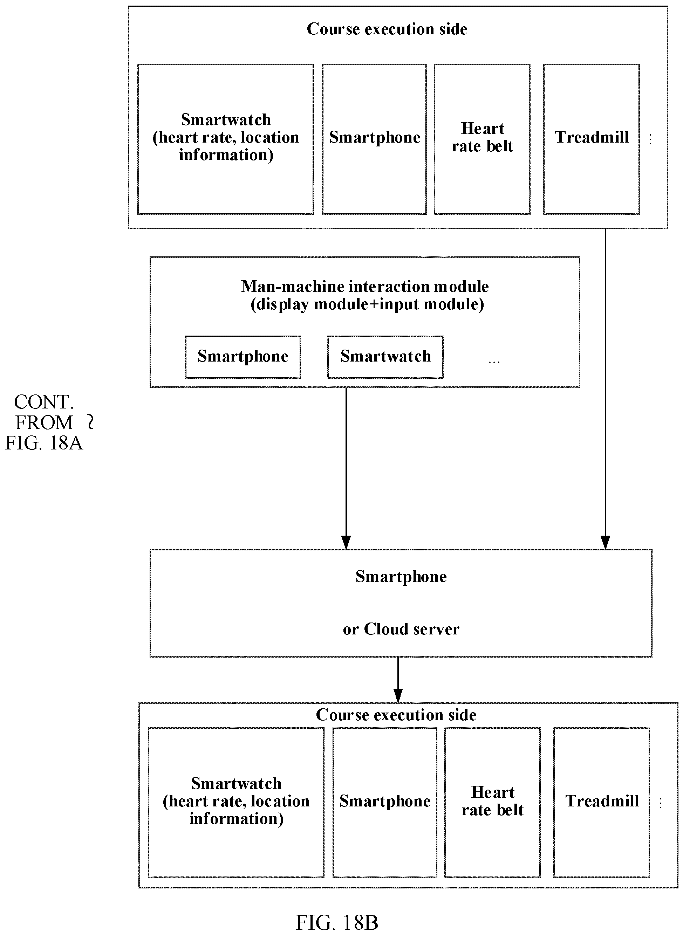

In this embodiment of this application, a procedure is executed by an electronic device. The electronic device includes but is not limited to a device that can execute a training plan generation task, such as a computer, a smartphone, a notebook computer, a tablet computer, a wearable intelligent device (such as a smart necklace, a smart watch, and a smart band), and a cloud server. FIG. 18A and FIG. 18B are an implementation flowchart of an adjustment method for a training plan according to an embodiment of this application. Details are as follows:
In S1802, a subjective parameter collection page is generated if it is detected that any training course in a training plan is completed, to determine, based on a feedback operation initiated by a user on the subjective parameter collection page, a subjective exercise parameter that corresponds to the any training course and that is of the user.

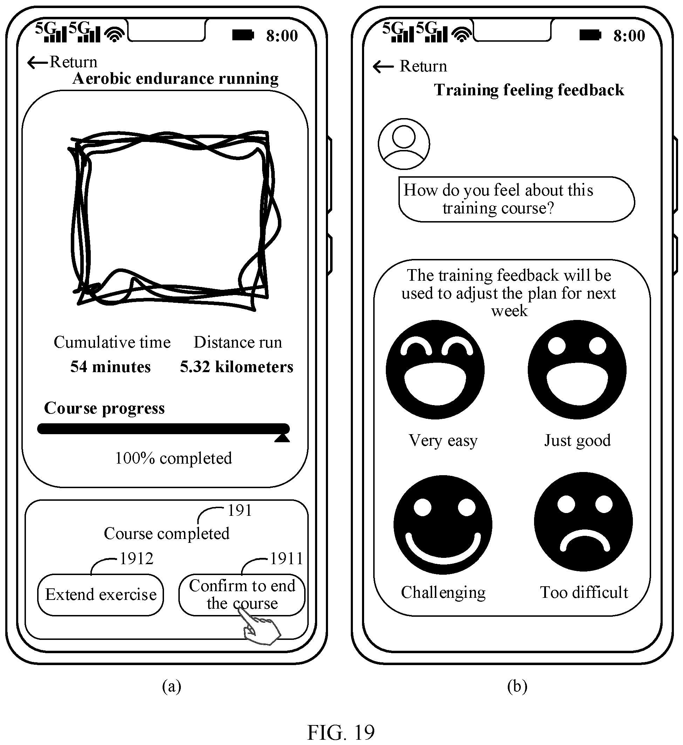

In this embodiment, after detecting that a user completes a training course, an electronic device may generate a subjective parameter collection page. For example, FIG. 19 is a schematic diagram of a subjective parameter collection page according to an embodiment of this application. Refer to (a) in FIG. 19. When detecting that a user completes a training course, an electronic device may pop up a prompt box 191 prompting that training ends. The prompt box displays a "confirm to end the course" control 1911 and an "extend exercise" control 1912. If it is detected that the user taps the control 1911, it is identified that the training course ends, and a page for collecting a subjective exercise parameter of the training course, namely, a subjective parameter collection page, as shown in (b) in FIG. 19, may be generated. The subjective parameter collection page includes controls corresponding to a plurality of optional subjective exercise parameters, which are separately "very easy", "just good", "challenging", and "too difficult". Subjective exercise intensity corresponding to the foregoing subjective exercise parameters increases sequentially. The user may select a corresponding subjective exercise parameter based on actual perception of training of the current course, and initiate a feedback operation on the subjective parameter collection page. If the user thinks that exercise intensity of the current training course is appropriate, the user may tap the "just good" control. The electronic device may determine a subjective exercise parameter based on the feedback operation of the user.

It should be noted that the subjective parameter collection page may be displayed on a display module of the electronic device, or may be displayed on a display module of another device. For example, a user performs course training on a treadmill. After detecting that the user completes a training course, the electronic device may send a generated subjective parameter collection page to another electronic device, or send a corresponding page generation instruction to another electronic device. In this example, the another electronic device is a treadmill, and the treadmill may display the subjective parameter collection page on a local display module, the user may initiate a feedback operation on the display module of the treadmill, and the treadmill sends a subjective exercise parameter corresponding to the feedback operation to the electronic device, so that the electronic device can obtain the subjective exercise parameter of the user.

Further, in another embodiment of this application, before S1802, the method may further include the following step:
In S1801, a corresponding training stress value obtained when the user performs training based on any training course in the training plan is obtained.

In this embodiment, after the electronic device generates the training plan, the user may perform training according to the training plan. The training plan may include a plurality of training times, and each training time may be associated with one or more training courses. The user may perform physical exercise based on training content in the training course. In a process in which the user performs physical exercise, there may be a corresponding training stress value based on exercise intensity. A larger training stress value indicates that the training course corresponds to higher exercise intensity. On the contrary, a smaller training stress value indicates that the training course corresponds to lower exercise intensity.

The training stress value may be determined based on a course difficulty level corresponding to a training course. For example, if a course difficulty level of a training course is 3, it may be determined that a training stress value of the user is 3. Certainly, the electronic device may further store a conversion relationship between a course difficulty level and a training stress value, and obtain, through calculation by using the conversion relationship, a training stress value corresponding to the training course. The electronic device may further determine the training stress value based on corresponding real-time data (namely, real-time exercise data) obtained when the user performs the training exercise.

If the electronic device determines the training stress value based on the real-time data of the user, S1801 may specifically include the following steps:
In S1801.1, corresponding real-time data is obtained when the user performs training based on any training course in the training plan.
In S1801.2, the training stress value corresponding to the training course is determined based on the real-time data.

In this embodiment, in a process of performing exercise, the user may obtain, by using the electronic device or another data collection device, corresponding real-time exercise data, namely, the foregoing real-time data, when the user performs training based on the training course. The real-time data may include a heart rate value in an exercise process and exercise duration.

In this embodiment, the real-time data includes heart rate values corresponding to a plurality of collection moments. In an exercise process of the user, the electronic device or another data collection device may obtain the heart rate value of the user in the exercise process at a preset time interval, and each heart rate value may correspond to one collection moment. When determining the training stress value of the user in a training course process, the electronic device may first determine, based on the heart rate value corresponding to each collection moment, exercise intensity corresponding to each moment. A manner of calculating the exercise intensity may be specifically determining the exercise intensity based on a ratio of an actual heart rate value of the user to an ideal heart rate value corresponding to the exercise item. After obtaining the exercise intensity corresponding to each collection moment through calculation, the electronic device may mark, in a preset coordinate system, coordinate points corresponding to collected heart rate values. Vertical coordinates of the coordinate points may be exercise intensity, and horizontal coordinates may be corresponding collection moments. Then, the electronic device may connect the coordinate points according to a sequence of the collection moments, to obtain an exercise intensity curve corresponding to the real-time data. The electronic device may perform integration on the exercise intensity curve in a time dimension, and use a value obtained through integration as the training stress value corresponding to the training course.

In a possible implementation, a manner in which the electronic device calculates the training stress value may further be: The electronic device determines average exercise intensity of the user based on heart rate values at a plurality of collection moments in the real-time data, and obtains, based on a product of exercise duration and the average exercise intensity, the training stress value corresponding to the training course.

In S1803, the training plan is adjusted based on the subjective exercise parameter, to generate an adjusted training plan.

In this embodiment, the electronic device may adjust the training plan based on the subjective exercise parameter of the user. For example, if the subjective exercise parameter indicates that it is difficult for the user to complete the training plan, difficulty of the training course in the training plan can be reduced. On the contrary, if the subjective exercise parameter indicates that it is easy for the user to complete the training plan, difficulty of the training course in the training plan can be increased, thereby ensuring that the training plan is consistent with training experience of the user.

Further, if the electronic device further obtains a corresponding training stress value when the user performs the training plan, S1803 may be specifically: adjusting the training plan based on the training stress value and the subjective exercise parameter, to generate an adjusted training plan.

In this embodiment, the electronic device may compare a training stress value of a same training course with a subjective exercise parameter, to determine whether actual exercise intensity of the training course is consistent with subjective perception of the user. Because each person has a personalized difference in feeling in an exercise process, a course difficulty level of a training course is set based on a common indicator. For example, a course difficulty level is determined based on feedback of a large quantity of users. However, the common indicator may not match personalized feelings of the user. To improve adaptation between a training plan and the user, improve training positiveness of the user, and cultivate a habit of continuous exercise of the user, the electronic device may adjust the training plan based on an objectively collected training stress value and the subjective exercise parameter, to obtain an adjusted training plan, to improve rationality of subsequent training course arrangement and a degree of conformity with the user.

Further, in another embodiment of this application, S1803 may specifically include the following types:
In S1803.1, if a preset adjustment trigger moment is reached, a plan adjustment direction is determined based on the subjective exercise parameter and training stress values corresponding to all the training courses completed before the adjustment trigger moment.

In this embodiment, when the adjustment trigger moment is not reached, the electronic device may store the obtained training stress value and the subjective exercise parameter in a memory, and does not adjust the training plan immediately after the training course is completed. As shown in (b) in FIG. 19, on a subjective parameter collection page generated after a user completes a training course, corresponding prompt information "training experience is used for adjustment of a plan in a next week" may be displayed. In other words, a subsequent training course is not adjusted immediately in this case, but the training plan is adjusted only when the user reaches a next week (namely, a preset adjustment trigger moment).

In this embodiment, the electronic device may preset an adjustment period for the training plan, and may determine a plurality of adjustment trigger moments based on period duration of the adjustment period. For example, if the adjustment period is one week (seven days), 00:00 on each Sunday may be set as an adjustment trigger moment, and a training course after a next week is adjusted by using a training stress value and a subjective exercise parameter that correspond to a training course completed in the current week.

In this embodiment, the electronic device may determine an adjustment direction for the training plan based on a training stress value and a subjective exercise parameter that correspond to each training course completed by the user. The adjustment direction includes: increasing training difficulty, reducing training difficulty, and maintaining training difficulty.

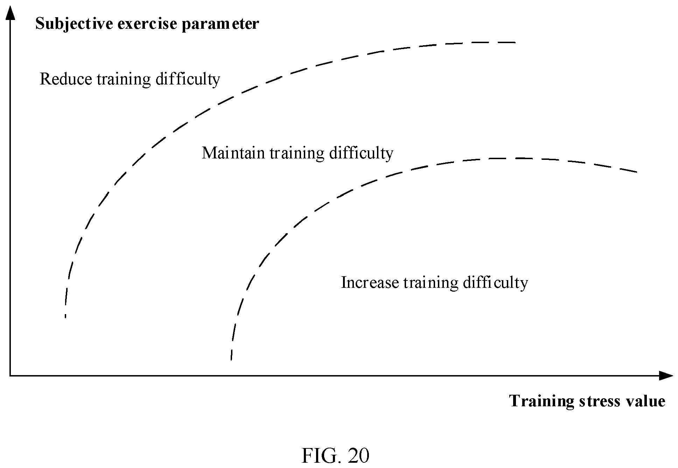

For example, FIG. 20 is a schematic diagram of a training plan adjustment direction according to an embodiment of this application. FIG. 20 is a reference coordinate system of an adjustment direction according to this embodiment. An electronic device may determine coordinates of a training course in the reference coordinate system based on a training stress value and a subjective exercise parameter of the training course. The coordinates may specifically fall into three different areas, which are respectively:
Case 1: For a same training course, if a training stress value corresponding to the training course is basically similar to a corresponding subjective exercise parameter, that is, actual exercise intensity of the training course basically matches subjective perception of a user, coordinates corresponding to the training course fall into an area corresponding to maintaining training difficulty, that is, the training plan does not need to be adjusted.
Case 2: For a same training course, if a training stress value corresponding to the training course is large, and a subjective exercise parameter fed back by the user is small, that is, actual exercise intensity of the training course is high, but the user thinks that the training course is easy to complete, that is, it indicates that the user has a capability of completing a training course with higher difficulty. In this case, coordinates corresponding to the training course fall into an area corresponding to increasing a difficulty, that is, a course difficulty level of the training course in the training plan needs to be increased.
Case 3: For a same training course, if a training stress value corresponding to the training course is small, and a subjective exercise parameter fed back by the user is large, that is, actual exercise intensity of the training course is low, but the user still thinks that it is difficult to complete the training course, that is, it indicates that it is challenging for the user to complete the training course, and the training course is a course with high difficulty for the user. In this case, coordinates corresponding to the training course fall into an area corresponding to reducing difficulty, that is, a course difficulty level of the training course in the training plan needs to be reduced.

In a possible implementation, because the user may complete a plurality of training courses when the adjustment trigger moment is met, the electronic device may mark coordinates of each training course in the reference coordinate system, and in addition to determining the adjustment direction of the training plan, may further determine an adjustment amplitude. If a plurality of training courses fall into an area for improving training difficulty, it indicates that a course difficulty level of the training course in the training plan is low, and an amplitude of being low is large. When the course is adjusted subsequently, the course difficulty level that matches the course may be increased. On the contrary, if a part of the training course falls into the area of maintaining training difficulty or even the area of reducing training difficulty, and the other part of the training course falls into the area of increasing training difficulty, it indicates that a course difficulty level is low, but an amplitude of being low is small. When the course is adjusted subsequently, a course difficulty level of the training course may be slightly increased, or a course difficulty level of a part of the training course is increased, and a course difficulty level of a part of the training course is maintained.

In a possible implementation, each training course corresponds to one course category. During adjustment, the electronic device may determine, based on a subjective exercise parameter and a training stress value that are of a completed training course and that correspond to a course category, an adjustment direction corresponding to the course category.

In S1803.2, a training course that is in the training plan and that is after the adjustment trigger moment is adjusted based on the plan adjustment direction, to obtain the adjusted training plan.

In this embodiment, the electronic device may adjust a subsequent training course in the training plan based on the plan adjustment direction, for example, increase a course difficulty level of the subsequent training course, or reduce a course difficulty level of the training course.

When generating a training plan, the electronic device may create corresponding training category libraries for different training phases. The training category library includes one or more course categories corresponding to the training phase, and one course category may include training courses with different course difficulty levels. Therefore, if a course difficulty level of a training course does not match a user, a training course with another course difficulty level may be selected from the training category corresponding to the training course, to replace the training course of the course category in the training plan.

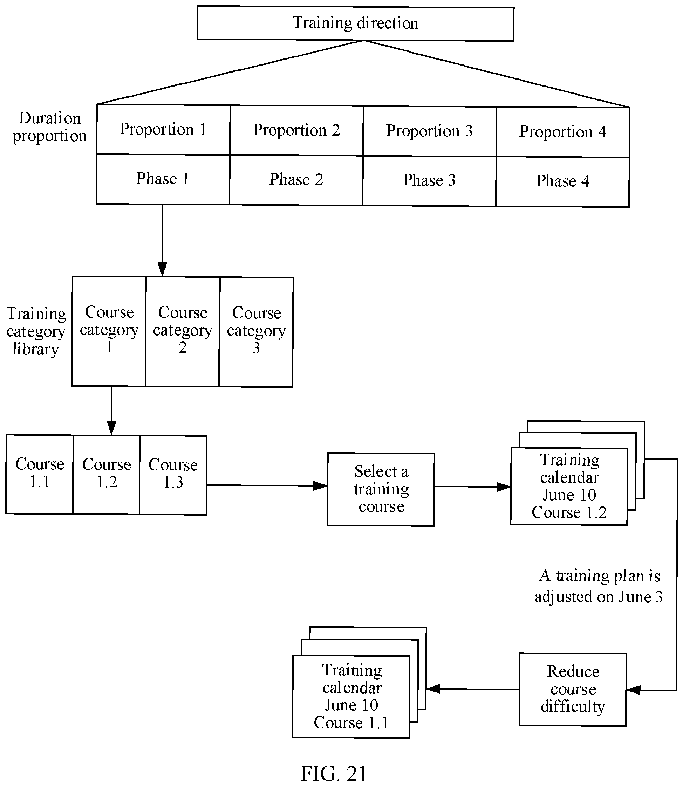

For example, FIG. 21 is a schematic diagram of adjustment of a training course according to an embodiment of this application. Refer to FIG. 21. Before adjustment, a training course for June 10 is a course 1.2, that is, a course difficulty level is 2. If the electronic device determines, on June 3, that a course difficulty level of a subsequent training course needs to be reduced, the electronic device needs to reduce course difficulty of a training course on a subsequent date, and identifies that a course category to which the training course for June 10 belongs is a course category 1. The course category 1 includes training courses of three course difficulty levels, and a training course whose course difficulty level is less than 2 is a course 1.1. In this case, the course 1.1 is used to replace the training course that has been arranged for June 10, and training courses of all training times are adjusted in the foregoing manner, so that adjusted training courses are obtained.

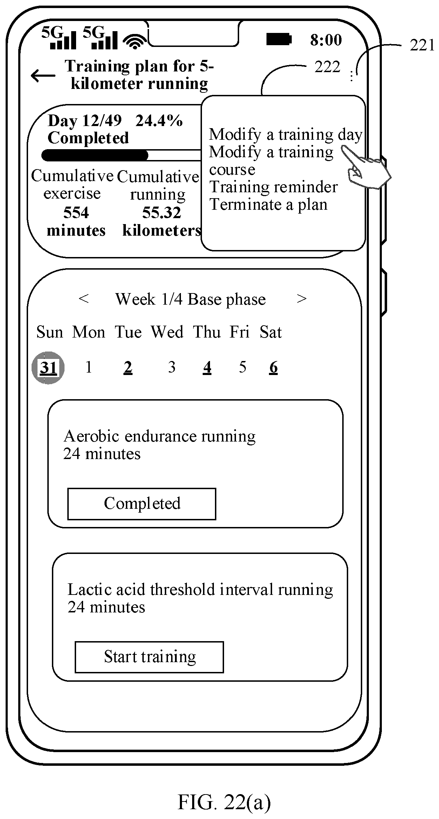

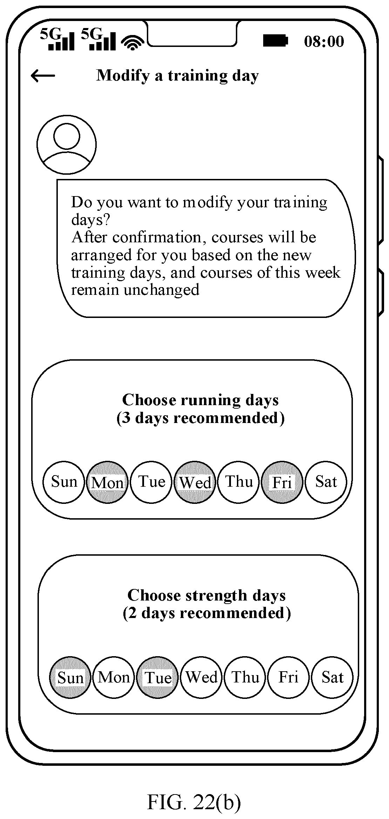

In a possible implementation, in addition to automatically adjusting the training plan in the foregoing manner, the electronic device may further receive an active modification request of a user, and adjust the training plan. For example, FIG. 22(a) to FIG. 22(c) are schematic diagrams of adjustment of a training plan according to an embodiment of this application. Refer to FIG. 22(a). A menu control 221 may be configured on a training course query interface of the electronic device. If it is detected that a user taps the menu control 221, a corresponding menu pop-up window 222 may be generated. The menu pop-up window 222 includes a plurality of options, which are respectively: "modify a training day", "modify a training course", "training reminder", and "terminate a plan". If the electronic device detects that the user taps the option of "modify a training day", a training day adjustment page is generated. As shown in FIG. 22(b), the user may set training dates corresponding to different training items on the training day adjustment page, for example, a training date of a running item and a training date of a strength item. The user may select a proper date based on a situation of the user. To improve stability of the training plan, the adjustment of the training day may take effect in a next week, or certainly, may take effect immediately.

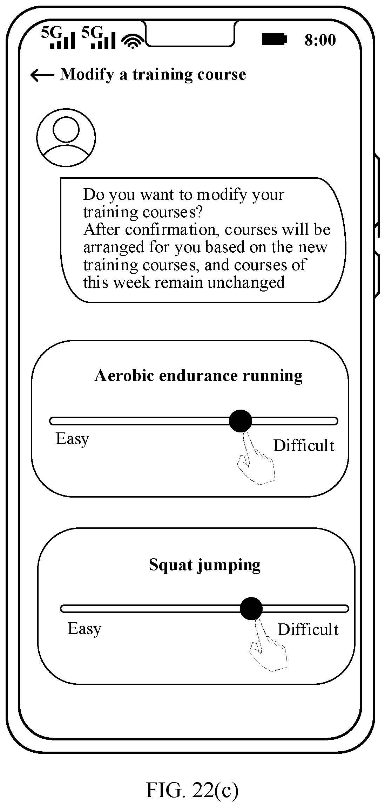

The electronic device detects that the user taps the option of "modify a training course", and may generate a training course adjustment page, as shown in FIG. 22(c). Specifically, each training course may display a current course difficulty level. The user may adjust, based to a situation of the user, a course difficulty level corresponding to each training course. The electronic device may select a training course of a corresponding course difficulty level from a course category corresponding to the training course for replacement. Similarly, the foregoing adjustment may be performed on the training plan at a next adjustment trigger moment, or may take effect immediately.

It can be learned from the foregoing that, according to the adjustment method for a training plan provided in this embodiment of this application, in addition to obtaining a training stress value of the user in an exercise process, a subjective exercise parameter of the user for a current training course may be further obtained after the training course ends, and a training plan is adjusted based on the purpose training stress value and the subjective exercise parameter related to subjective perception of the user. This can improve accuracy of training plan adjustment and further improve a requirement of the user for personalized setting of the training plan.

It should be understood that sequence numbers of the steps do not mean an execution sequence in embodiments. The execution sequence of the processes needs to be determined based on functions and internal logic of the processes, and should not constitute any limitation on the implementation processes of embodiments of this application.

### Embodiment 4

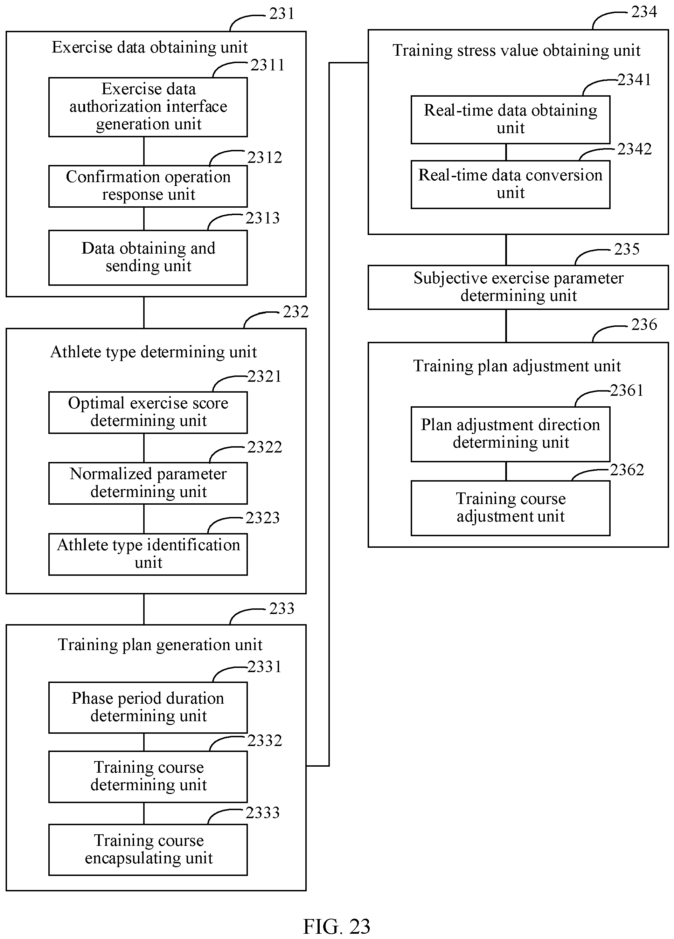

With reference to the generation methods for a training plan described in Embodiment 1 and Embodiment 2 and the adjustment method for a training plan described in Embodiment 3, FIG. 23 is a block diagram of a structure of a generation apparatus for a training plan according to an embodiment of this application. For ease of description, only a part related to this embodiment of this application is shown.

Refer to FIG. 23. The generation apparatus for a training plan includes:
an exercise data obtaining unit 231, configured to obtain a plurality of pieces of exercise data of a user;
an athlete type determining unit 232, configured to determine, based on the plurality of pieces of exercise data, an athlete type corresponding to the user; and
a training plan generation unit 233, configured to: determine a training direction of the user based on the athlete type, and generate a training plan matching the training direction.

Optionally, the obtained plurality of pieces of exercise data include exercise data of a plurality of exercise items, and the athlete type determining unit 232 includes:
an optimal exercise score determining unit 2321, configured to determine, based on the exercise data associated with each exercise item, an optimal exercise score corresponding to the exercise item;
a normalized parameter determining unit 2322, configured to import the optimal exercise score into a normalization algorithm corresponding to the exercise item, and determining a normalized parameter corresponding to the exercise item; and
an athlete type identification unit 2323, configured to determine the athlete type of the user based on a normalized parameter of each exercise item.

Optionally, the optimal exercise score determining unit 2321 includes:
an ideal heart rate determining unit, configured to determine an ideal heart rate corresponding to the exercise item, where the ideal heart rate is specifically a corresponding heart rate of a user in a full-effort exercise state;
an intensity coefficient determining unit, configured to determine, based on an actual heart rate in the exercise data and the ideal heart rate, an intensity coefficient associated with the exercise data; and
an optimal exercise score calculation unit, configured to determine, based on the intensity coefficient and an actual exercise score, the optimal exercise score corresponding to the exercise item.

Optionally, the exercise data obtaining unit 231 includes:
an exercise data authorization interface generation unit 2311, configured to generate an exercise data authorization interface that includes at least one optional data source;
a confirmation operation response unit 2312, configured to determine a target data source from the optional data source in response to a confirmation operation performed by the user on the exercise data authorization interface; and
a data obtaining and sending unit 2313, configured to obtain the exercise data from the target data source.

Optionally, the generation apparatus for a training plan further includes:
a training report display unit, configured to display a training report corresponding to the user, where the training report includes the training plan and a descriptive paragraph about the athlete type and the training direction.

Optionally, the training plan generation unit 233 includes:
a phase period duration determining unit 2331, configured to determine, based on the training direction, phase period duration of each pre-divided training phase in a preset plan template;
a training course determining unit 2332, configured to: determine a course category associated with the training phase, and configure a training course that belongs to the course category for each training time in the training phase, where the training time is determined based on the phase period duration of the training phase; and
a training course encapsulating unit 2333, configured to generate the training plan based on training courses corresponding to training times of all training phases.

Optionally, the training course determining unit 2332 includes:
a training category library configuration unit, configured to configure a training category library for the training phase based on the course category associated with the training phase;
a training course framework determining unit, configured to determine, based on the phase period duration of the training phase and the training category library, a training course framework corresponding to the training phase, where the training course framework is used to determine the training time included in the training phase and a course difficulty level corresponding to each training time; and
a training course selecting unit, configured to select, for each training time from the training category library, the training course matching the course difficulty level.

Optionally, the phase period duration determining unit 2331 includes:
a plan template obtaining unit, configured to obtain the plan template corresponding to a training purpose of the user, where the training purpose is preset by the user, and the plan template includes a plurality of training phases obtained through pre-division;
a duration proportion determining unit, configured to determine, based on the training direction, a duration proportion of each training phase; and
a total training duration allocation unit, configured to determine the phase period duration of the training phase based on the duration proportion of the training phase and preset total training duration.

Optionally, the generation apparatus for a training plan further includes:
a setting interface display unit, configured to generate a training purpose setting interface, where the training purpose setting interface includes a purpose setting area of at least one exercise item;
a setting operation response unit, configured to determine, in response to a setting operation of the user in each purpose setting area, a target value corresponding to each exercise item; and
a training purpose determining unit, configured to obtain the training purpose based on the target value of each exercise item.

Optionally, the generation apparatus for a training plan further includes:
a subjective exercise parameter determining unit 235, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit 236, configured to adjust the training plan based on the subjective exercise parameter, to generate an adjusted training plan.

Optionally, the generation apparatus for a training plan further includes:
a training stress value obtaining unit 234, configured to obtain a corresponding training stress value when the user performs training based on any training course in the training plan; and
a training plan adjustment unit 236, specifically configured to adjust the training plan based on the training stress value and the subjective exercise parameter, to generate the adjusted training plan.

Optionally, the training stress value obtaining unit 234 includes:
a real-time data obtaining unit 2341, configured to obtain corresponding real-time data when the user performs training based on any training course in the training plan; and
a real-time data conversion unit 2342, configured to determine, based on the real-time data, the training stress value corresponding to the training course.

Optionally, the training plan adjustment unit 236 includes:
a plan adjustment direction determining unit 2361, configured to: if a preset adjustment trigger moment is reached, determine a plan adjustment direction based on the subjective exercise parameter and training stress values corresponding to all the training courses completed before the adjustment trigger moment; and
a training course adjustment unit 2362, configured to adjust, based on the plan adjustment direction, a training course that is in the training plan and that is after the adjustment trigger moment, to obtain the adjusted training plan.

Therefore, the generation apparatus for a training plan provided in this embodiment of this application determines an athlete type of the user by obtaining exercise data of a user in a historical exercise process, identifies, based on the athlete type, a training direction corresponding to training performed by the user, and generates a training plan that matches the training direction, so that a training plan that adapts to an exercise capability of the user can be generated. Because an individual parameter of the user cannot directly reflect the exercise ability of the user, the training plan generated based on the individual parameter may not match the exercise ability of the user. However, the exercise data that is collected and that is in a historical exercise activity of the user can reflect an actual exercise ability of the user. The athlete type of the user is determined based on performance of the user in historical exercise, so that a matching degree between the training plan and the actual exercise ability of the user can be improved. In this way, the generated training plan can be used to guide the user to perform exercise and training more pertinently, and accuracy of the generated training plan is improved.

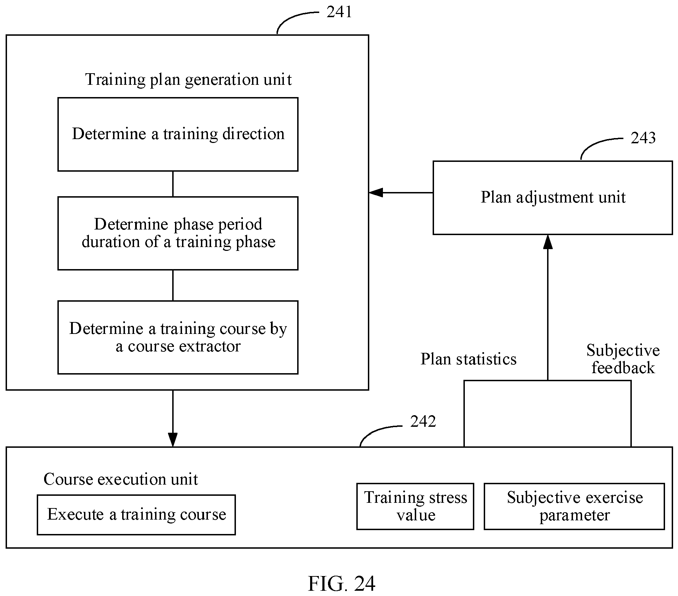

Further, with reference to the generation methods for a training plan in Embodiment 1 and Embodiment 2, and adjustment method for a solution training plan provided in Embodiment 3, FIG. 24 is a block diagram of a structure of a generation apparatus for a training plan according to another embodiment of this application. Refer to FIG. 24. The generation apparatus for a training plan may specifically include a training plan generation unit 241, a course execution unit 242, and a plan adjustment unit 243.

The training plan generation unit 241 is configured to perform the generation methods for a training plan provided in Embodiment 1 and Embodiment 2, that is, determine a training direction based on exercise data of a user, and determine, based on the training direction, phase period duration of each training phase in a plan template. The training plan generation unit 241 may further include a course extractor. After the phase period duration corresponding to each training phase is determined, a corresponding training course may be selected for each training phase by using the course extractor, to generate a training plan. The training plan generation unit 242 may be located in a smartphone on a user side, or may be located in a cloud server.

The course execution unit 242 may receive each training course delivered by the training plan generation unit 241, and output each training course through the course execution unit 242, for example, prompt a user to execute the training course in a reminder manner, or display, through a display module, a guidance video corresponding to the training course. Then, the course execution unit 242 may further obtain a corresponding training stress value and subjective exercise parameter when the user executes the training course. The course execution unit 242 may be located in a smartphone, a smartwatch, a treadmill, a smart band, or the like on the user side.

The plan adjustment unit 243 is specifically configured to adjust the training plan based on the training stress value and the subjective exercise parameter that are fed back by the course execution unit 242, and feed back an adjusted training plan to the training plan generation unit 241. The plan adjustment unit 243 may be located in a smartphone on the user side, or may be located in a server on a cloud.

### Embodiment 5

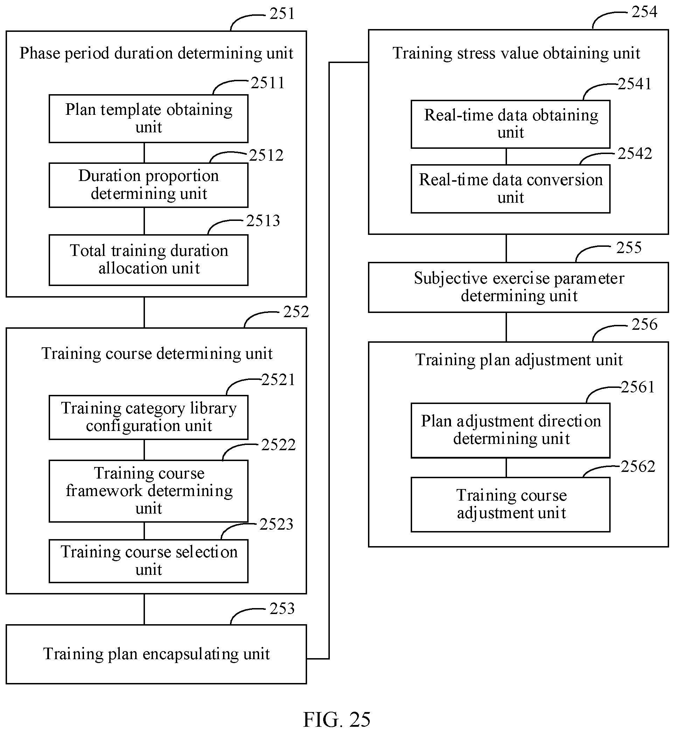

With reference to the generation methods for a training plan described in the foregoing Embodiment 2 and the adjustment method for a training plan described in the foregoing Embodiment 3, FIG. 25 is a block diagram of a structure of a generation apparatus for a training plan according to an embodiment of this application. For ease of description, only a part related to this embodiment of this application is shown.

Refer to FIG. 25. The generation apparatus for a training plan includes:
a phase period duration determining unit 251, configured to determine, based on a training direction of a user, phase period duration of each pre-divided training phase in a preset plan template;
a training course determining unit 252, configured to: determine a course category associated with the training phase, and configure a training course that belongs to the course category for each training time in the training phase, where the training time is determined based on the phase period duration of the training phase; and
a training plan encapsulating unit 253, configured to generate the training plan based on training courses corresponding to training times of all training phases.

Optionally, the training course determining unit 252 includes:
a training category library configuration unit 2521, configured to configure a training category library for the training phase based on the course category associated with the training phase;
a training course framework determining unit 2522, configured to determine, based on the phase period duration of the training phase and the training category library, a training course framework corresponding to the training phase, where the training course framework is used to determine the training time included in the training phase and a course difficulty level corresponding to each training time; and
a training course selecting unit 2523, configured to select, for each training time from the training category library, the training course matching the course difficulty level.

Optionally, the phase period duration determining unit 251 includes:
a plan template obtaining unit 2511, configured to obtain the plan template corresponding to a training purpose of the user, where the training purpose is preset by the user, and the plan template includes a plurality of training phases obtained through pre-division;
a duration proportion determining unit 2512, configured to determine, based on the training direction, a duration proportion of each training phase; and
a total training duration allocation unit 2513, configured to determine the phase period duration of the training phase based on the duration proportion of the training phase and preset total training duration.

Optionally, the generation apparatus for a training plan further includes:
a setting interface display unit, configured to generate a training purpose setting interface, where the training purpose setting interface includes a purpose setting area of at least one exercise item;
a setting operation response unit, configured to determine, in response to a setting operation of the user in each purpose setting area, a target value corresponding to each exercise item; and
a training purpose determining unit, configured to obtain the training purpose based on the target value of each exercise item.

Optionally, the generation apparatus for a training plan further includes:
a subjective exercise parameter determining unit 255, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit 256, configured to adjust the training plan based on the subjective exercise parameter, to generate an adjusted training plan.

Optionally, the generation apparatus for a training plan further includes:
a training stress value obtaining unit 254, configured to obtain a corresponding training stress value when the user performs training based on any training course in the training plan; and
a training plan adjustment unit 256, specifically configured to adjust the training plan based on the training stress value and the subjective exercise parameter, to generate the adjusted training plan.

Optionally, the training stress value obtaining unit 254 includes:
a real-time data obtaining unit 2541, configured to obtain corresponding real-time data when the user performs training based on any training course in the training plan; and
a real-time data conversion unit 2542, configured to determine, based on the real-time data, the training stress value corresponding to the training course.

Optionally, the training plan adjustment unit 256 includes:
a plan adjustment direction determining unit 2561, configured to: if a preset adjustment trigger moment is reached, determine a plan adjustment direction based on the subjective exercise parameter and training stress values corresponding to all the training courses completed before the adjustment trigger moment; and
a training course adjustment unit 2562, configured to adjust, based on the plan adjustment direction, a training course that is in the training plan and that is after the adjustment trigger moment, to obtain the adjusted training plan.

Therefore, the generation apparatus for a training plan provided in this embodiment of this application may also adjust phase period duration of each training phase in a training plan based on a training direction of a user. Different training phases correspond to different training focuses. Therefore, a corresponding course category may be associated, and a corresponding training course is configured for each training time based on a course category corresponding to the training phase, to generate a training plan that matches the training direction of the user. In this way, the training plan can improve a capability of the user in the training direction in a targeted manner, and the training plan is not determined based on an individual parameter of the user. This improves adaptation between the training plan and the user and improves pertinence of a training process.

### Embodiment 6

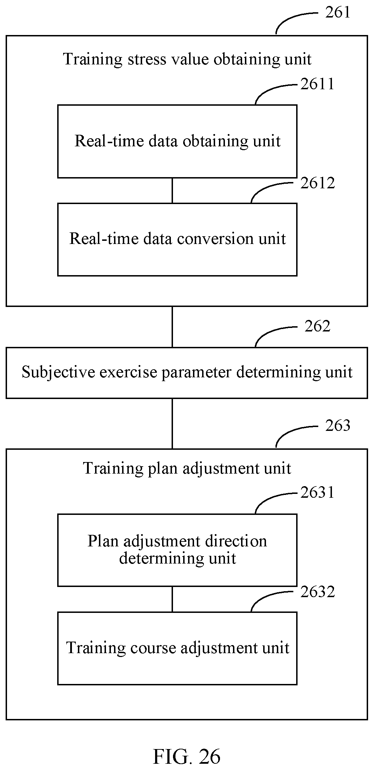

Corresponding to the adjustment method for a training plan described in Embodiment 3, FIG. 26 is a block diagram of a structure of an adjustment apparatus for a training plan according to an embodiment of this application. For ease of description, only a part related to this embodiment of this application is shown.

Refer to FIG. 26. The adjustment apparatus for a training plan includes:
a subjective exercise parameter determining unit 262, configured to generate a subjective parameter collection page if it is detected that any training course in the training plan is completed, to determine, based on a feedback operation initiated by the user on the subjective parameter collection page, a subjective exercise parameter that corresponds to the any training course and that is of the user; and
a training plan adjustment unit 263, configured to adjust the training plan based on the subjective exercise parameter, to generate an adjusted training plan.

Optionally, the adjustment apparatus for a training plan further includes:
a training stress value obtaining unit 261, configured to obtain a corresponding training stress value when the user performs training based on any training course in the training plan; and
a training plan adjustment unit 263, specifically configured to adjust the training plan based on the training stress value and the subjective exercise parameter, to generate the adjusted training plan.

Optionally, the training stress value obtaining unit 261 includes:
a real-time data obtaining unit 2611, configured to obtain corresponding real-time data when the user performs training based on any training course in the training plan; and
a real-time data conversion unit 2612, configured to determine, based on the real-time data, the training stress value corresponding to the training course.

Optionally, the training plan adjustment unit 263 includes:
a plan adjustment direction determining unit 2631, configured to: if a preset adjustment trigger moment is reached, determine a plan adjustment direction based on the subjective exercise parameter and training stress values corresponding to all the training courses completed before the adjustment trigger moment; and
a training course adjustment unit 2632, configured to adjust, based on the plan adjustment direction, a training course that is in the training plan and that is after the adjustment trigger moment, to obtain the adjusted training plan.

Therefore, in addition to obtaining a training stress value of the user in an exercise process, the adjustment apparatus for a training plan provided in this embodiment of this application may further obtain a subjective exercise parameter of the user for a current training course after the training course ends, and adjust a training plan based on the purpose training stress value and the subjective exercise parameter related to subjective perception of the user. This can improve accuracy of training plan adjustment and further improve a requirement of the user for personalized setting of the training plan.

The generation method for a training plan provided in the embodiments of this application may be applied to an electronic device such as a mobile phone, a tablet computer, a wearable device, an in-vehicle device, an augmented reality (augmented reality, AR)/virtual reality (virtual reality, VR) device, a notebook computer, an ultra-mobile personal computer (ultra-mobile personal computer, UMPC), a netbook, a personal digital assistant (personal digital assistant, PDA), a smartwatch, a smart band, or a server. A specific type of the electronic device is not limited in embodiments of this application.

For example, the electronic device may be a station (STATION, ST) in a WLAN, or may be a cellular phone, a cordless phone, a session initiation protocol (Session Initiation Protocol, SIP) phone, a wireless local loop (Wireless Local Loop, WLL) station, a personal digital processing (Personal Digital Assistant, PDA) device, a handheld device with a wireless communication function, a computing device, or another processing device connected to a wireless modem, a computer, a laptop computer, a handheld communication device, a handheld computing device, and/or another device used for communication in a wireless system and a next-generation communication system, for example, a mobile terminal in a 5G network or a mobile terminal in a future evolved public land mobile network (Public Land Mobile Network, PLMN) network.

By way of example, and not limitation, when the terminal device is a wearable device, the wearable device may alternatively be a generic term for wearable devices such as glasses, gloves, watches, clothes, and shoes that are developed based on intelligent design of daily wearing by using wearable technologies. The wearable device is a portable device that is directly worn on a body or integrated into clothes or accessories of a user, and collects biometric feature data of the user, for example, a heart rate value of the user, by attaching to the user. The wearable device is not only a hardware device, but also implements a powerful function through software support, data exchange, and cloud interaction. In a broad sense, wearable intelligent devices include devices that are with comprehensive functions, large sizes, and can implement full or partial functions without depending on a smartphone, such as a smart watch or smart glasses, and devices that focus only on a specific type of application functions and need to be used with another device such as a smartphone, such as various smart bands and smart jewelry that include an unlockable touchscreen.

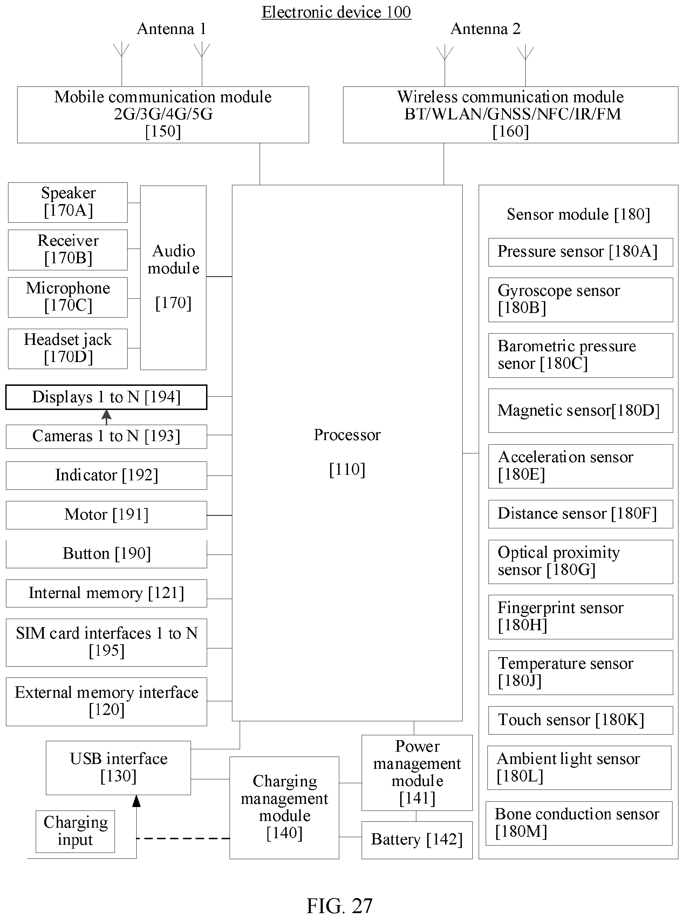

FIG. 27 is a schematic diagram of a structure of an electronic device 100.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include an exercise sensor. The exercise sensor may include a pressure sensor 180A, a gyroscope sensor 180B, and an acceleration sensor 180E. The sensor module 180 may further include a barometric pressure sensor 180C, a magnetic sensor 180D, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors. The processor may be configured to perform operations of S202 and S203 in Embodiment 1, operations of S2021 to S2023, and operations of S2021' and S2022', may be further configured to perform operations of S1301 to S 1303, operations of S1301.1 to S1301.3, and operations of S1302.1 to S1302.3 in Embodiment 2, and may be further configured to perform operations of S1803, and operations of S1803.1 and S1803.2 in Embodiment 3.

The controller may generate an operation control signal based on an instruction operation code and a time sequence signal, to complete control of instruction reading and instruction execution.

A memory may be further disposed in the processor 110, and is configured to store instructions and data, for example, exercise data of a user, a generated training plan, and a created training course library. In some embodiments, the memory in the processor 110 is a cache memory. The memory may store an instruction or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a bidirectional synchronization serial bus, and includes a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be used for audio communication.

The UART interface is a universal serial data bus, and is configured to perform asynchronous communication. The bus may be a two-way communications bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

It may be understood that an interface connection relationship between modules shown in this embodiment of this application is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

A wireless communication function of the electronic device 100 may be implemented through the antenna 1, the antenna 2, the mobile communication module 150, the wireless communication module 160, the modem processor, the baseband processor, and the like.

The wireless communication module 160 may provide a wireless communication solution that is applied to the electronic device 100, and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave by the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communications technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The internal memory 121 may be configured to store computer-executable program code. The executable program code includes instructions. The internal memory 121 may include a program storage area and a data storage area. The program storage area may store an operating system, an application required by at least one function (for example, a wireless connection function, a vibration wave generation function, an adjustment program for a training plan, and a generation program for a training plan that are provided in embodiments of this application), and the like. The data storage area may store data (for example, audio data and a phone book) created in a process of using the electronic device 100, and may further store exercise data obtained from a target data source, a generated training plan, a plan template, a training course framework, a training category library, a training course, user information entered by a user, a fed-back subjective exercise parameter in embodiments of this application, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The pressure sensor 180A is configured to sense a stress signal, and can convert the stress signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines stress intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In some embodiments, touch operations that are performed in a same touch position but have different touch operation intensity may correspond to different operation instructions. For example, when a touch operation whose touch operation intensity is less than a first stress threshold is performed on an SMS message application icon, an instruction for viewing an SMS message is performed. When a touch operation whose touch operation intensity is greater than or equal to the first stress threshold is performed on the SMS message application icon, an instruction for creating a new SMS message is performed.

The gyroscope sensor 180B may be configured to determine a moving posture of the electronic device 100. In some embodiments, an angular velocity of the electronic device 100 around three axes (namely, axes x, y, and z) may be determined through the gyroscope sensor 180B. The gyroscope sensor 180B may be configured to implement image stabilization during photographing. For example, when the shutter is pressed, the gyroscope sensor 180B detects an angle at which the electronic device 100 shakes, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device, and is used in an application such as switching between a landscape mode and a portrait mode or a pedometer.

The distance sensor 180F is configured to measure a distance. The electronic device 100 may measure the distance in an infrared manner or a laser manner. In some embodiments, in a photographing scenario, the electronic device 100 may measure a distance through the distance sensor 180F to implement quick focusing.

The button 190 includes a power button, a volume button, and the like. The button 190 may be a mechanical button, or may be a touch button. The electronic device 100 may receive a key input, and generate a key signal input related to a user setting and function control of the electronic device 100.

The motor 191 may generate a vibration prompt, for example, a vibration signal in this application. The motor 191 may be configured to provide an incoming call vibration prompt and a touch vibration feedback. For example, touch operations performed on different applications (for example, photographing and audio playback) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

A software system of the electronic device 100 may use a layered architecture, an eventdriven architecture, a microkernel architecture, a micro service architecture, or a cloud architecture. In an embodiment of this application, an Android system with a layered architecture is used as an example to describe the software structure of the electronic device 100.

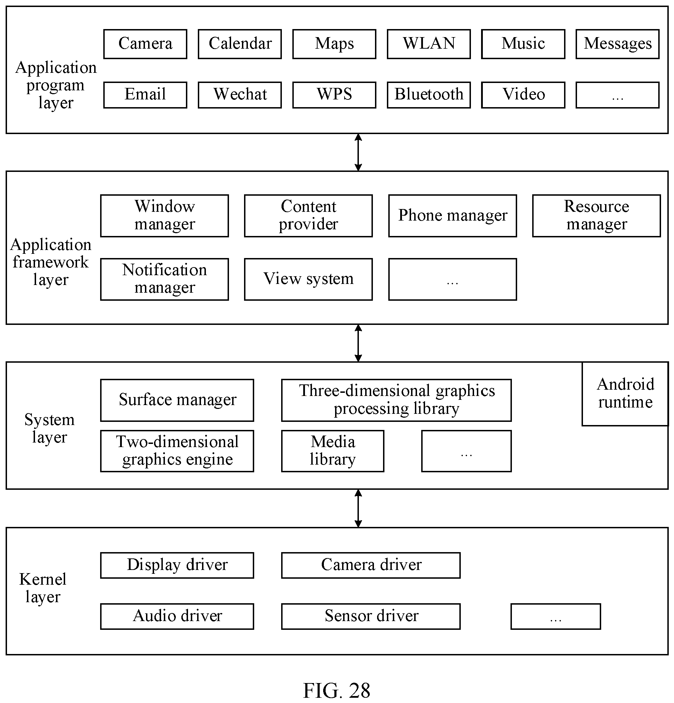

FIG. 28 is a block diagram of a software structure of an electronic device 100 according to an embodiment of this application.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 28, the application package may include applications such as Camera, Calendar, Map, Mailbox, WLAN, Bluetooth, Music, Video, SMS, WeChat, and WPS. Further, the application package may further include a training management program provided in embodiments of this application, and a training plan is generated and adjusted by using the training management program.

The application framework layer provides an application programming interface (application programming interface, API) and a programming framework for an application at the application layer. The application framework layer includes some predefined functions.

As shown in FIG. 28, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, contacts, and the like.

The view system includes visual controls such as a control for displaying a text and a control for displaying an image. The view system may be configured to construct an application. A display interface may include one or more views. For example, a display interface including an SMS message notification icon may include a text display view and an image display view, and may further include an exercise data authorization interface, a training purpose setting interface, a subjective parameter collection page, a training report interface, and the like that are provided in embodiments of this application.

The phone manager is configured to provide a communication function for the electronic device 100, for example, management of a call status (including answering, declining, or the like).

The resource manager provides various resources such as a localized character string, an icon, an image, a layout file, a video file for an application, and exercise data obtained from a target data source.

The notification manager enables an application to display notification information in a status bar, and may be configured to convey a notification message. The notification manager may automatically disappear after a short pause without requiring a user interaction. For example, the notification manager is used to notify a download completion, give a message notification, notify authorization information of exercise data, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is displayed in the status bar, an announcement is given, the electronic device vibrates, or the indicator light blinks.

The Android runtime includes a kernel library and a virtual machine. The Android runtime is responsible for scheduling and management of the Android system.

The kernel library includes two parts: a function that needs to be called in Java language and a kernel library of Android.

The application layer and the application framework layer run on the virtual machine. The virtual machine executes java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG-4, H.264, MP3, AAC, AMR, JPG, and PNG.

The three-dimensional graphics processing library is used to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

With reference to a scenario in which exercise data is obtained from a target data source, the following describes a working procedure of software and hardware of the electronic device 100 by using an example.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. For example, the touch operation is a touch and tap operation, and a control corresponding to the tap operation is a "determine" control 502 in the exercise data authorization interface (a selected target data source is a smartwatch). The training management application invokes an interface of the application framework layer to start a Bluetooth communication module, and then invokes the kernel layer to start a Bluetooth driver in the wireless communication module 160, establishes a wireless connection to the smartwatch by using Bluetooth in the wireless communication module 160, obtains exercise data from the smartwatch, and transmits the exercise data to the processor 110 through an I2C interface between the wireless communication module 160 and the processor 110, and the processor 110 may generate a training plan of the user based on the received exercise data.

It should be noted that content such as information exchange between the foregoing apparatuses/units and the execution processes thereof is based on a same concept as the method embodiments of this application. For specific functions and technical effects of the content, refer to the method embodiments. Details are not described herein again.

A person skilled in the art may clearly understand that, for the purpose of convenient and brief description, division into the foregoing functional units or modules is merely used as an example for description. In an actual application, the foregoing functions may be allocated to different functional units or modules for implementation based on a requirement. That is, an inner structure of the apparatus is divided into different functional units or modules to implement all or some of the functions described above. Functional units and modules in embodiments may be integrated into one processing unit, each of the units may exist alone physically, or two or more units may be integrated into one unit. The integrated unit may be implemented in a form of hardware, or may be implemented in a form of a software functional unit. In addition, specific names of the functional units and modules are merely for ease of distinguishing between the functional units and modules, but are not intended to limit the protection scope of this application. For a specific working process of the units or modules in the foregoing system, refer to a corresponding process in the method embodiments. Details are not described herein again.

An embodiment of this application further provides a network device. The network device includes at least one processor, a memory, and a computer program that is stored in the memory and that can run on the at least one processor. When executing the computer program, the processor implements steps in any one of the foregoing method embodiments.

An embodiment of this application further provides a computer-readable storage medium. The computer-readable storage medium stores a computer program. When the computer program is executed by a processor, the steps in the foregoing method embodiments may be implemented.

An embodiment of this application provides a computer program product. When the computer program product runs on a mobile terminal, the mobile terminal is enabled to implement the steps in the foregoing method embodiments when executing the computer program product.

When the integrated unit is implemented in the form of the software functional unit and sold or used as an independent product, the integrated unit may be stored in a computer-readable storage medium. Based on such an understanding, all or some of the procedures of the method in embodiments of this application may be implemented by a program instructing related hardware. The computer program may be stored in a computer-readable storage medium. When the computer program is executed by the processor, steps in the foregoing method embodiments may be implemented. The computer program includes computer program code. The computer program code may be in a source code form, an object code form, an executable file form, some intermediate forms, or the like. The computer-readable medium may include at least any entity or apparatus that can carry the computer program code to a photographing apparatus/electronic device, a recording medium, a computer memory, a read-only memory (ROM, Read-Only Memory), a random access memory (RAM, Random Access Memory), an electrical carrier signal, a telecommunications signal, and a software distribution medium, for example, a USB flash drive, a removable hard disk, a magnetic disk, or an optical disk. In some jurisdictions, the computer-readable medium cannot be an electrical carrier signal or a telecommunication signal according to legislation and patent practices.

In the foregoing embodiments, descriptions of embodiments have respective focuses. For a part that is not described in detail in an embodiment, refer to related descriptions in another embodiment.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

In embodiments provided in this application, it should be understood that the disclosed apparatus/network device and method may be implemented in other manners. For example, the described apparatus/network device embodiment is merely an example. For example, division into the modules or units is merely logical function division and may be other division in an actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

The foregoing embodiments are merely intended to describe the technical solutions of this application, but are not to limit this application. Although this application is described in detail with reference to the foregoing embodiments, a person of ordinary skill in the art should understand that a person of ordinary skill in the art may still make modifications to the technical solutions described in the foregoing embodiments or make equivalent replacements to some technical features thereof, without departing from the spirit and scope of the technical solutions of embodiments of this application, and these modifications and replacements shall fall within the protection scope of this application.

## Claims

1. A generation method for a training plan, comprising:
obtaining a plurality of pieces of exercise data of a user;
determining, based on the plurality of pieces of exercise data, an athlete type corresponding to the user; and
determining a training direction of the user based on the athlete type, and generating a training plan matching the training direction.

2. The generation method according to claim 1, wherein the obtained plurality of pieces of exercise data comprise exercise data of a plurality of exercise items, and the determining, based on the plurality of pieces of exercise data, an athlete type corresponding to the user comprises:
determining, based on the exercise data associated with each exercise item, an optimal exercise score corresponding to the exercise item;
importing the optimal exercise score into a normalization algorithm corresponding to the exercise item, and determining a normalized parameter corresponding to the exercise item; and
determining the athlete type of the user based on a normalized parameter of each exercise item.

3. The generation method according to claim 2, wherein the determining, based on the exercise data associated with each exercise item, an optimal exercise score corresponding to the exercise item comprises:
determining an ideal heart rate corresponding to the exercise item, wherein the ideal heart rate is specifically a corresponding heart rate of a user in a full-effort exercise state;
determining, based on an actual heart rate in the exercise data and the ideal heart rate, an intensity coefficient associated with the exercise data; and
determining, based on the intensity coefficient and an actual exercise score, the optimal exercise score corresponding to the exercise item.

4. The generation method according to claim 1, wherein the obtaining a plurality of pieces of exercise data of a user comprises:
generating an exercise data authorization interface that comprises at least one optional data source;
determining a target data source from the optional data source in response to a confirmation operation performed by the user on the exercise data authorization interface; and
obtaining the exercise data from the target data source.

5. The generation method according to claim 1, wherein after the determining a training direction of the user based on the athlete type, and generating a training plan matching the training direction, the method further comprises:
displaying a training report corresponding to the user, wherein the training report comprises the training plan and a descriptive paragraph about the athlete type and the training direction.

6. The generation method according to any one of claims 1 to 5, wherein the determining a training direction of the user based on the athlete type, and generating a training plan matching the training direction comprises:
determining, based on the training direction, phase period duration of each pre-divided training phase in a preset plan template;
determining a course category associated with the training phase, and configuring a training course that belongs to the course category for each training time in the training phase, wherein the training time is determined based on the phase period duration of the training phase; and
generating the training plan based on training courses corresponding to training times of all training phases.

7. The generation method according to claim 6, wherein the determining a course category associated with the training phase, and configuring a training course that belongs to the course category for each training time in the training phase comprises:
configuring a training category library for the training phase based on the course category associated with the training phase;
determining, based on the phase period duration of the training phase and the training category library, a training course framework corresponding to the training phase, wherein the training course framework is used to determine the training time comprised in the training phase and a course difficulty level corresponding to each training time; and
selecting, for each training time from the training category library, the training course matching the course difficulty level.

8. The generation method according to claim 6, wherein the determining, based on the training direction, phase period duration of each pre-divided training phase in a preset plan template comprises:
obtaining the plan template corresponding to a training purpose of the user, wherein the training purpose is preset by the user, and the plan template comprises a plurality of training phases obtained through pre-division;
determining, based on the training direction, a duration proportion of each training phase; and
determining the phase period duration of the training phase based on the duration proportion of the training phase and preset total training duration.

9. The generation method according to claim 8, wherein before the obtaining the plan template corresponding to a training purpose of the user, the method further comprises:
generating a training purpose setting interface, wherein the training purpose setting interface comprises a purpose setting area of at least one exercise item;
determining, in response to a setting operation of the user in each purpose setting area, a target value corresponding to each exercise item; and
obtaining the training purpose based on the target value of each exercise item.

10. The generation method according to any one of claims 1 to 9, wherein after the determining a training direction of the user based on the athlete type, and generating a training plan matching the training direction, the method further comprises:
generating a subjective parameter collection page if it is detected that any training course in the training plan is completed, to determine, based on a feedback operation initiated by the user on the subjective parameter collection page, a subjective exercise parameter that corresponds to the any training course and that is of the user; and
adjusting the training plan based on the subjective exercise parameter, to generate an adjusted training plan.

11. The generation method according to claim 10, wherein before the generating a subjective parameter collection page if it is detected that any training course in the training plan is completed, the method further comprises:
obtaining a corresponding training stress value when the user performs training based on the any training course; and
correspondingly, the adjusting the training plan based on the subjective exercise parameter, to generate an adjusted training plan comprises:
adjusting the training plan based on the training stress value and the subjective exercise parameter, to generate the adjusted training plan.

12. The generation method according to claim 11, wherein the obtaining a corresponding training stress value when the user performs training based on the any training course comprises:
obtaining corresponding real-time data when the user performs training based on any training course in the training plan; and
determining, based on the real-time data, the training stress value corresponding to the training course.

13. The generation method according to claim 11, wherein the adjusting the training plan based on the training stress value and the subjective exercise parameter, to generate the adjusted training plan comprises:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

14. A generation method for a training plan, comprising:
determining, based on a training direction of a user, phase period duration of each pre-divided training phase in a preset plan template;
determining a course category associated with the training phase, and configuring a training course that belongs to the course category for each training time in the training phase, wherein the training time is determined based on the phase period duration of the training phase; and
generating the training plan based on training courses corresponding to training times of all the training phases.

15. The generation method according to claim 14, wherein the determining a course category associated with the training phase, and configuring a training course that belongs to the course category for each training time in the training phase comprises:
configuring a training category library for the training phase based on the course category associated with the training phase;
determining, based on the phase period duration of the training phase and the training category library, a training course framework corresponding to the training phase, wherein the training course framework is used to determine the training time comprised in the training phase and a course difficulty level corresponding to each training time; and
selecting, for each training time from the training category library, the training course matching the course difficulty level.

16. The generation method according to claim 15, wherein the determining, based on a training direction of a user, phase period duration of each pre-divided training phase in a preset plan template comprises:
obtaining the plan template corresponding to a training purpose of the user, wherein the training purpose is preset by the user, and the plan template comprises a plurality of training phases obtained through pre-division;
determining, based on the training direction, a duration proportion of each training phase; and
determining the phase period duration of the training phase based on the duration proportion of the training phase and preset total training duration.

17. The generation method according to claim 16, wherein before the obtaining the plan template corresponding to a training purpose of the user, the method further comprises:
generating a training purpose setting interface, wherein the training purpose setting interface comprises a purpose setting area of at least one exercise item;
determining, in response to a setting operation of the user in each purpose setting area, a target value corresponding to each exercise item; and
obtaining the training purpose based on the target value of each exercise item.

18. The generation method according to any one of claims 14 to 17, wherein after the generating the training plan based on training courses corresponding to training times of all the training phases, the method further comprises:
generating a subjective parameter collection page if it is detected that any training course in the training plan is completed, to determine, based on a feedback operation initiated by the user on the subjective parameter collection page, a subjective exercise parameter that corresponds to the any training course and that is of the user; and
adjusting the training plan based on the subjective exercise parameter, to generate an adjusted training plan.

19. The generation method according to claim 18, before the generating a subjective parameter collection page if it is detected that any training course in the training plan is completed, the method further comprises:
obtaining a corresponding training stress value when the user performs training based on the any training course; and
correspondingly, the adjusting the training plan based on the subjective exercise parameter, to generate an adjusted training plan comprises:
adjusting the training plan based on the training stress value and the subjective exercise parameter, to generate the adjusted training plan.

20. The generation method according to claim 19, wherein the obtaining a corresponding training stress value when the user performs training based on the any training course comprises:
obtaining corresponding real-time data when the user performs training based on any training course in the training plan; and
determining, based on the real-time data, the training stress value corresponding to the training course.

21. The generation method according to claim 19, wherein the adjusting the training plan based on the training stress value and the subjective exercise parameter, to generate the adjusted training plan comprises:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

22. An adjustment method for a training plan, comprising:
generating a subjective parameter collection page if it is detected that any training course in a training plan is completed, to determine, based on a feedback operation initiated by a user on the subjective parameter collection page, a subjective exercise parameter that corresponds to the any training course and that is of the user; and
adjusting the training plan based on the subjective exercise parameter, to generate an adjusted training plan.

23. The adjustment method according to claim 22, before the generating a subjective parameter collection page if it is detected that any training course in a training plan is completed, the method further comprises:
obtaining a corresponding training stress value when the user performs training based on the any training course; and
correspondingly, the adjusting the training plan based on the subjective exercise parameter, to generate an adjusted training plan comprises:
adjusting the training plan based on the training stress value and the subjective exercise parameter, to generate the adjusted training plan.

24. The adjustment method according to claim 23, wherein the obtaining a corresponding training stress value when the user performs training based on the any training course comprises:
obtaining corresponding real-time data when the user performs training based on any training course in the training plan; and
determining, based on the real-time data, the training stress value corresponding to the training course.

25. The adjustment method according to claim 23 or 24, wherein the adjusting the training plan based on the training stress value and the subjective exercise parameter, to generate the adjusted training plan comprises:
if a preset adjustment trigger moment is reached, determining a plan adjustment direction based on the subjective exercise parameter and training stress values corresponding to all the training courses completed before the adjustment trigger moment; and
adjusting, based on the plan adjustment direction, a training course that is in the training plan and that is after the adjustment trigger moment, to obtain the adjusted training plan.

26. An electronic device, comprising a memory, a processor, and a computer program that is stored in the memory and that can be run on the processor, wherein when executing the computer program, the processor implements the method according to any one of claims 1 to 25.

27. A computer-readable storage medium, wherein the computer-readable storage medium stores a computer program, and when the computer program is executed by a processor, a computer is enabled to implement the method according to any one of claims 1 to 25.
